# EUROPEAN PATENT APPLICATION

(11) **EP 4 382 533 A1**
(43) Date of publication of application: **12.06.2024**
(21) Application number: 22383185.0
(22) Date of filing: 05.12.2022
(51) Int. Cl.: C07K 14/46, A61K 38/19, A61K 39/12, C07K 14/005, C07K 14/52, C12N 15/62

(54) **PROTEIN NANOPELLETS FOR FISH OR TELEOST AND CRUSTACEAN VACCINATION**

(71) Applicant: Universitat Autònoma de Barcelona, 08193 Bellaterra (Cerdanyola del Vallès) (ES)
(72) Inventor: ROHER ARMENTIA, Nerea, 08400 Granollers (Barcelona) (ES); ROJAS PEÑA, Mauricio Eduardo, 0805 Barcelona (Barcelona) (ES); ACEITUNO ABARZUA, Patricia Eliana, 08005 Barcelona (Barcelona) (ES)

(57) **Abstract**

The present invention relates to polypeptides comprising the amino acid sequence of an interferon or a fragment thereof, the amino acid sequence of a viral antigen or a fragment thereof and, optionally, a linker between them. It also discloses nanopellets comprising the said polypeptides with a particle diameter from 400 nm to 1500 nm measured with electron microscopy, as well as their use in immunogenic compositions and vaccines to immunize fish or teleost and crustacean against several virus diseases.

## Description

### Technical Field

Present invention belongs to the field of fusion proteins. In particular, the invention relates to a fusion protein with immunization activity. The fusion protein of the invention is particularly useful in the immunization against viral infections in fish or teleost and crustaceans by means of oral vaccination.

### Background Art

According to the Food and Agriculture Organization (FAO), disease outbreaks cost the global aquaculture industry around US$6 billion per year and represent the major farm-level risk. In particular, viral diseases are a major concern, and there is a continuous emergence of them. Considering the gravity and frequency of aquatic animals' disease outbreaks, new strategies for their prevention, detection, and management are urgently needed.

Among viral disease, spring viremia of carp (SVC) is an infectious viral disease common to carp (*Cyprinus carpio*) and other cyprinid fish species (including zebrafish (*Danio rerio*)*,* koi (*Cyprinus carpio koi*)*,* silver carp (*Hypophthalmichthys molitrix*), goldfish (*Carassius auratus*) or grass carp (*Ctenopharyngodon idella*) among others, caused by the SVC virus (SVCV). SVCV is a member of the family Rhabdoviridae, with negative-sense ssRNA encoding five proteins-nucleoprotein (N), phosphoprotein (P), matrix protein (M), glycoprotein (G), and RNA-dependent polymerase (L). The expansion of the geographical and host Infection range of SVCV poses a major threat not only to the fish farms and ornamental fish industries, but also to wild fish populations. SVC outbreaks can reach 90% of mortality in juvenile fish.

Among the strategies, vaccination is one of the most promising ones. But it needs to optimize efficacy, while taking into account production and administration costs, environmental risks, and compliance with legislation.

Regarding SVCV, therapeutic or prophylactic approaches to combat SVCV have been ineffective and no commercial vaccine is currently available.

The traditional approach is based on the use of inactivated or attenuated viral vaccines, which are commercially available for certain viral diseases that cause high mortality. However, their main drawbacks are that not all fish viruses are readily culturable in cells; that the administration via injection or immersion for juveniles is expensive, thus only deemed for the most valuable species; and the risk of possible reversion to virulence and environmental spread.

New approaches are thus being sought. Among them, recombinant DNA vaccines have achieved promising results against certain viruses, but they also raise safety and environmental issues regarding genetically modified organisms and their administration by intramuscular injection is costly and difficult to perform on juveniles, as well as causing stress and injury to fish, so it is only worth it in high added value species.

An alternative vaccine approach is the use of recombinant protein viral antigens. The main advantage of subunit vaccines is they are safe: there is no risk of DNA integration into the host, reversion, or invasion. Their main drawbacks are the stability and half-life of recombinant proteins in vivo; and the lack of pathogen associated molecular patterns (PAMPS) that the immune system utilizes to recognize pathogens via pattern recognition receptors (PRRs) thus needing adjuvants with co-stimulatory activity to enhance the magnitude of the immune response.

Oral delivery would be the most practical, cheap, and least stressful delivery method. However, the harsh gastric environment promotes antigen breakdown. So, immunorelevant epitopes need to be protected against gastrointestinal pH, which is particularly low in carnivorous fish, as well as digestive enzymes within the tract.

Aiming to protect the recombinant protein antigens from rapid degradation when inside the animal, different encapsulation techniques such as alginate and chitosan are being tested. Such strategies add an extra production step which increases the cost and makes it expensive for low market value species.

At this end, viral protein antigens nanostructured as bacterial inclusion bodies (IBs) were presented as a novel approach to prophylactic design, enhancing the stability of antigenic proteins while maintaining functionality. IBs are highly stable, tuneable, nanoscale protein particles which can penetrate cells, while retaining significant biological activity, as demonstrated by rescue studies.

They can be designed to bear the antigenic protein or epitopes of interest and provide a slow release of functional protein over time. The attractiveness of IBs as a fish or teleost and crustacean prophylactic is manifold. Their stability at gastrointestinal pH allows administrating the antigen orally through the feed, avoiding the necessity for vaccine encapsulation and the cost and stress to fish associated with injection. Production in *Escherichia coli* is achieved in bulk with a simple enzymatic and mechanical purification procedure which minimizes costs. This straightforward process implies that the IBs carry over fragments of bacterial lipopolysaccharide, peptidoglycans, and nucleic acids as impurities, but which are known adjuvants and immunomodulators of fish. The IB vehicle, a carrier and viral antigen as one biomaterial, elicit both an innate and adaptive immune response against the target virus in fish or teleost and crustacean. Additionally, IBs' stability under lyophilizing conditions and over a range of temperatures indicates their potential as a practical farm product with a lasting shelf life, avoiding the cold chain.

The potential of IBs have been demonstrated as an immunostimulant for fish, by nanostructuring recombinant cytokines TNF-a and CCL4 and testing them in a bacterial Infection model in zebrafish. In addition, uptake of the TNF-a IB by intestinal cells was demonstrated *in vivo* in rainbow trout via oral intubation.
For nanostructured viral antigens as IBs for use in fish food, the term "NanoPellets" (NPs) was coined.

In a recent study three recombinant viral antigenic proteins (viral capsid protein 2 (VP2) from infectious pancreatic necrosis virus (IPNV), an Aquabirnavirus; the glycoprotein G of viral haemorrhagic septicaemia virus (VHSV), a Novirhabdovirus; and the C coat protein of viral nervous necrosis virus (VNNV), a Betanodavirus) were produced as nanostructured biomaterials with view to use in orally delivered prophylaxis. The NanoPellets were successfully taken up *in vitro* in Zebra Fish Liver Cell Line (ZFL) and *in vivo* in zebrafish (*Danio rerio*) via oral administration. They stimulated an antiviral innate immune response both in ZFL and rainbow trout (*Oncorhynchus mykiss*) head kidney macrophage cell cultures (RT-HKM) cells. Being therefore candidates for immunostimulants (see Thwaite R, Ji J, Torrealba O, Coil J, Sabés M, Villaverde A and Roher N (2018) Protein Nanoparticles Made of Recombinant Viral Antigens: A Promising Biomaterial for Oral Delivery of Fish Prophylactics. Front. Immunol. 9:1652. https://doi.org/10.3389/fimmu.2018.01652).

All in all, NanoPellets present two main characteristics that makes them a very promising administration strategy for oral and immersion vaccine development for fish and shellfish as they protect the subunits from their high susceptibility to degradation and contain contaminants (in very small amounts) such as endotoxin, nucleic acids or peptidoglycans that can in turn work also as immunostimulators because all of them are well known PAMPs, that the immune system utilizes to recognize pathogens via pattern recognition receptors.

But, despite the intrinsic presence of PAMPs, additional adjuvants with co-stimulatory activity to enhance the magnitude of the immune response are required.

Therefore, there is still a need for enhancing the viral antigen protein nanostructure inclusion bodies prophylactic potential.

### Summary of Invention

Inventors have surprisingly found that a particular fusion polypeptide including an antigenic protein module and in-vaccine adjuvant in the same sequence which surprisingly allowed to obtain a functional and stable active system for the oral immunization of fishes or teleost and certain crustacean.

A common feature of anti-viral responses against different virus is the release of interferons (IFNs). And IFNs are known to be pivotal to mount an antiviral defense both in innate and adaptive immunity against virus, in particular, IFN gamma is especially important in potentiating the antiviral defenses. Although IFNs can be used for treatment of different diseases, their stability remained the main obstacle that hindered the utilization of recombinant proteins as therapeutics. IFNs failed to show the expected bioactivity in vivo due to degradation, and also to the tendency to aggregate irreversibly under mild denaturing conditions. Additional studies with recombinant IFNs administration to fish by oral or immersion route failed, due to their extreme lability, in the induction of the expression of Mx proteins, which are the only IFN-induced proteins known to posse antiviral properties. Mx proteins are dynamin-like large GTPases, and GTPase activity is required for their antiviral activity. MxA recognizes incoming viral proteins as well as newly synthesized viral proteins in the cytoplasm of infected cells and inhibits transport of viral proteins into the nucleus, thus blocking early steps in the viral life cycle.

Surprisingly, the inventors found that nanostructured polypeptides including IFN combined with an antigenic module remained stable and functional even under the harshest conditions of pH and temperature.

Thus, a first aspect of the invention is a polypeptide comprising the amino acid sequence of an interferon or a fragment thereof, the amino acid sequence of a viral antigen or a fragment thereof and, optionally, a linker between them.

As indicated above, and as will be illustrated in the examens below, one or more polypeptides could be nanostructured, forming a NanoPellet, presenting surprisingly high stability, even in harsh conditions, of both the antigenic module (i.e., viral antigen or fragment thereof) and the in-vaccine adjuvant (i.e., interferon or fragment thereof), and they showed enhanced immunogenic activity. This is a great advance as improves the potential of the NanoPellets administration approach, showing their high versatility and that they could include other modules such as ligands for specific molecules or receptors, that would provide a plethora of extra functions to the recombinant antigenic protein.

Thus, in a second aspect, the invention relates to a protein NanoPellet comprising one or more of the polypeptides as defined in the first aspect, and with a particle diameter from 400 nm to 1500 nm, measured by electron microscopy.

As shown in the examples, the inventors found that nanopellets, containing the polypeptides defined in the first aspect, administered by immersion were efficiently uptake by zebrafish larvae cells and elicited a strong antiviral response. Therefore, due to the capability of promoting an enhanced immunogenic reaction, the polypeptide as well as the protein NanoPellet are used in immunogenic compositions.

Thus, a third aspect of the invention is an immunogenic composition comprising one or more polypeptides as defined in the first aspect, and/or the protein nanopellet as defined in the second aspect.

The inventors vaccinated adult zebrafish with the nanopellets as defined in the second aspect and infected the animals with spring viremia carp virus. The results surprisingly showed excellent levels of survival compared with non-vaccinated and control fish, and the degrees of protection were even different from previous attempts using a DNA vaccine engineered with a plasmid that drives the expression of the SVCV-G protein.

Thus, a fourth aspect of the invention is a vaccine comprising the immunogenic composition as defined above, and a pharmaceutically acceptable excipient and/or carrier.

A fifth aspect of the invention is a polypeptide as defined in the first aspect and/or the protein nanopellet as defined in the second aspect, and/or the immunogenic composition as defined in the third aspect, and/or the vaccine as defined in the fourth aspect, for use in therapy. In other words, they are for use as a medicament.

Another (sixth) aspect of the invention is a food composition comprising a polypeptide, and/or a protein nanopellet, and/or the immunogenic composition, and/or the vaccine, as defined above. The food composition comprises at least one edible ingredient.

The invention also provides as an additional aspect a polynucleotide encoding the polypeptide as defined in the first aspect.

Also herewith disclosed is a vector comprising the polynucleotide encoding the polypeptide as defined in the first aspect.

Yet another aspect is a cell system comprising the polypeptide and/or the vector, both as defined above.

More precisely, the invention provides a host cell which is transformed or transfected with the polynucleotide and/or the vector as defined above, and it also provides a cell culture comprising said host cell.

### Brief Description of Drawings

Figure 1A. Characterization of nanostructured SVCV^{NP} proteins. (i) Field Emission Scanning Electron Microscopy images (FESEM). The scale line reference's units are micrometers (µm).
   Size distribution histograms (n=60) of length (x-axis represents the length in micrometers (µm)) (ii) and width (x-axis represents the width in micrometers (µm)) (iii) measurements of SVCV^{NP}.
Figure 1B. Characterization of nanostructured SVCV-IFN^{NP} proteins. (i) Field Emission Scanning Electron Microscopy images (FESEM). The scale line reference's units are micrometers (µm).
   Size distribution histograms measurements of SVCV-IFN^{NP} (n=60): of (ii) length (x-axis represents the length in micrometers (µm)); and (iii) width (x-axis represents the width in micrometers (µm)) (iii).

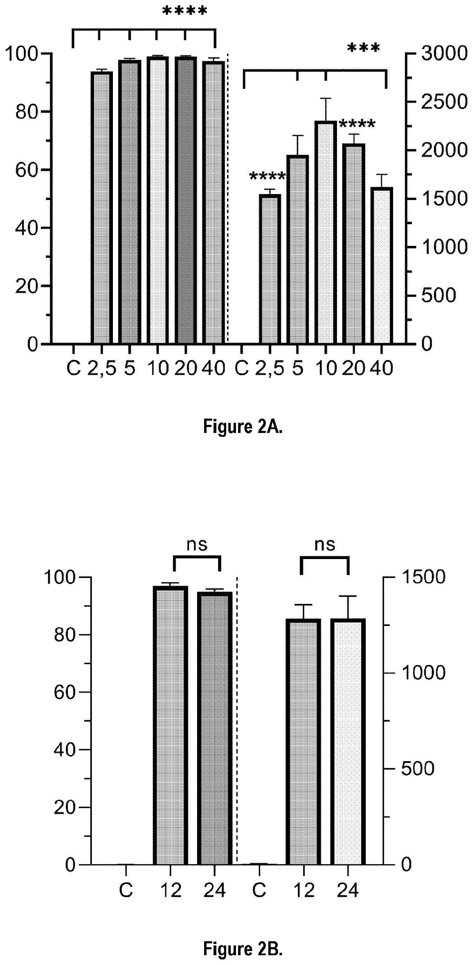

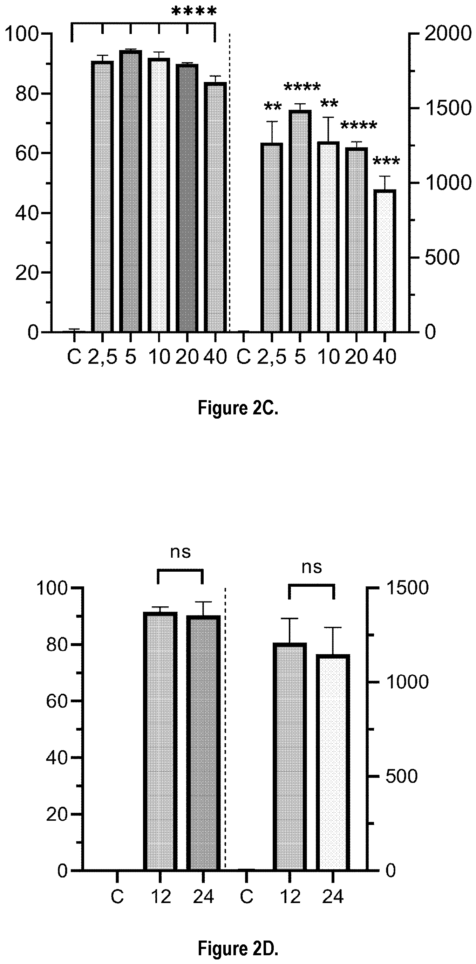

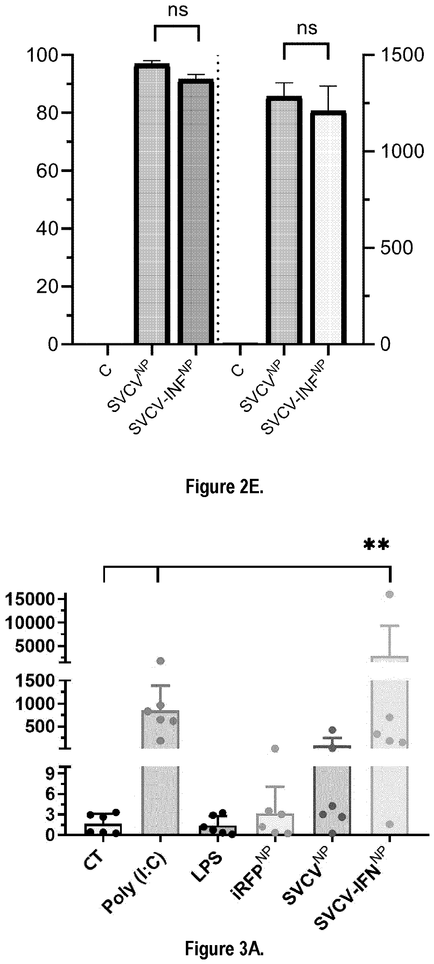

Figure 2. Uptake of SVCV and SVCV-IFN nanopellets by ZFL cells. (A) SVCV^{NP} dose response (x-axis represents Dose in pg/ml; left y-axis represents percentage of Fluorescent cells (%); right y-axis represents Mean Fluorescent Intensity (MFI); "C" stands for Control); (B) SVCV time course (x-axis represents Time in hours (h); left y-axis represents percentage of Fluorescent cells (%); right y-axis represents Mean Fluorescent Intensity (MFI); "ns" stands for Non-Significant; "C" stands for Control); (C) SVCV-IFN^{NP} dose response (x-axis represents Dose in µg/ml (h); left y-axis represents percentage of Fluorescent cells (%); right y-axis represents Mean Fluorescent Intensity (MFI); "C" stands for Control);; (D) SVCV-IFN^{NP} time course (x-axis represents Time in hours (h); left y-axis represents percentage of Fluorescent cells (%); right y-axis represents Mean Fluorescent Intensity (MFI); "ns" stands for Non-Significant; "C" stands for Control); and (E) Comparative uptake of SVCV^{NP} and SVCV-IFN^{NP} at 10 µg/ml for 12 h (left y-axis represents percentage of Fluorescent cells (%); right y-axis represents Mean Fluorescent Intensity (MFI); "ns" stands for Non-Significant; "C" stands for Control). For dose response assays 2,5 - 40 µg/ml of fluorescently labeled SVCV and SVCV-IFN were added to ZFL cells and incubated for 12 h and the uptake evaluated by cytometry. For time course 10 µg/ml of fluorescently labeled SVCV and SVCV-IFN were added to ZFL cells and incubated for 12 and 24 h. Differences between means were analyzed by a one-way ANOVA with Dunnett's multiple comparisons test, treatments versus control. Significance levels **p* < 0.05; ***p <* 0.01; ****p* < 0.001; *****p <* 0.0001.

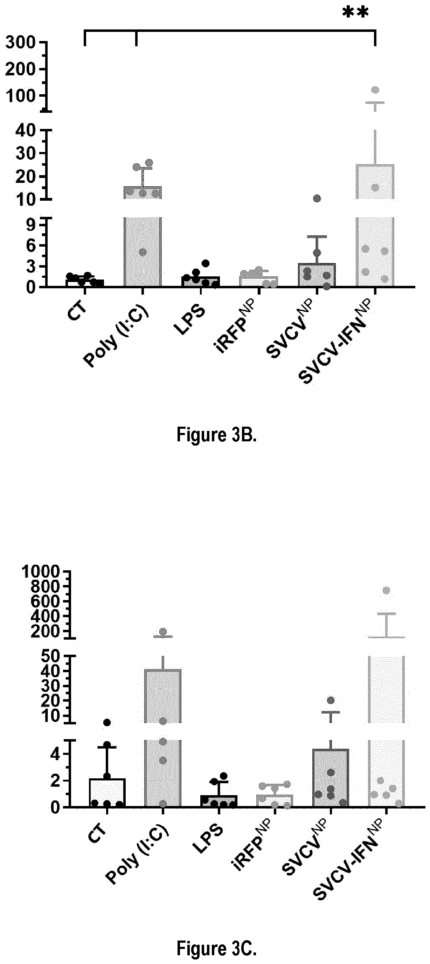

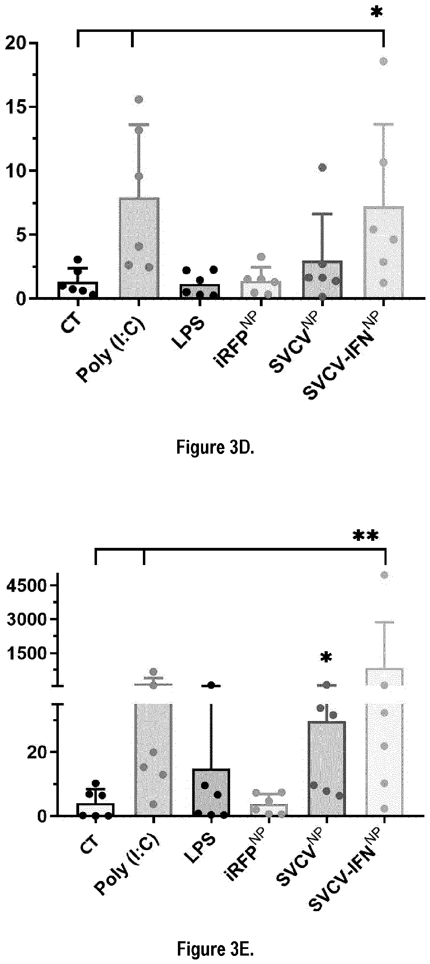

Figure 3. Gene expression analysis of ZFL treated with SVCV^{NP} and SVCV-IFN^{NP}: (A) vig1, (B) mx, (C) ifngr1, (D) Imp2, and (E) ccl4. ZFL cells were incubated as follow: unstimulated control cells, 10 µg/ml of Poly(I:C) and LPS, 10 µg/ml of SVCV, 10 µg/ml of SVCV-IFN and 10 µg/ml of near-infrared fluorescent protein (from now on: iRFP^{NP}) as immunogenically irrelevant control. Data are mean ± SD (n=6). Statistical differences between treatments and controls were analyzed by unpair non parametric Mann-Whitney test. Significance levels **p* < 0.05; ***p* < 0.01. CT stands for Control. Poly(I:C) means Polyinosinic:polycytidylic acid. The y-axis represents the Relative Expression.
Figure 4. Biodistribution *in vivo* in zebrafish larvae. (A) Control, (B) SVCV^{NP}, and (C) SVCV-IFN^{NP}. Uptake in larvae. Zebrafish larvae (3 dpf) were immersed with 10 µg/ml of fluorescent SVCV^{NP} and SVCV-IFN^{NP} for 48 h. E3 medium with PBS were used as control. (i) Transmitted image, (ii) fluorescent image and (iii) merged image. G stands for Gastrointestinal tract. P stands for Pancreas.
Figure 5. SVCV^{NP} and SVCV-IFN^{NP} administration protects from SVC virus *in vivo* Infection.
   (A) Representative fish images of SVC virus clinical signs.
   (B) Cumulative mortality rate of zebrafish after SVCV challenge. Fish were vaccinated with 5 µg/fish of different antigens (iRFP^{NP}, SVCV^{NP}, SVCV-IFN^{NP}) by intraperitoneal injection. Thirty days post-vaccination fish were challenged with SVCV (4 × 10⁴ TCID50/ml). Number of dead fish in each group was counted daily. The x-axis represents the days after trials. The y-axis represents the cumulative mortality (%). The line of squares represents the control SVCV; the line of circles represents the iRFP^{NP}; the filled triangles line represents the SVCV^{NP}; the empty inverted triangles line represents the SVCV-IFN^{NP}.

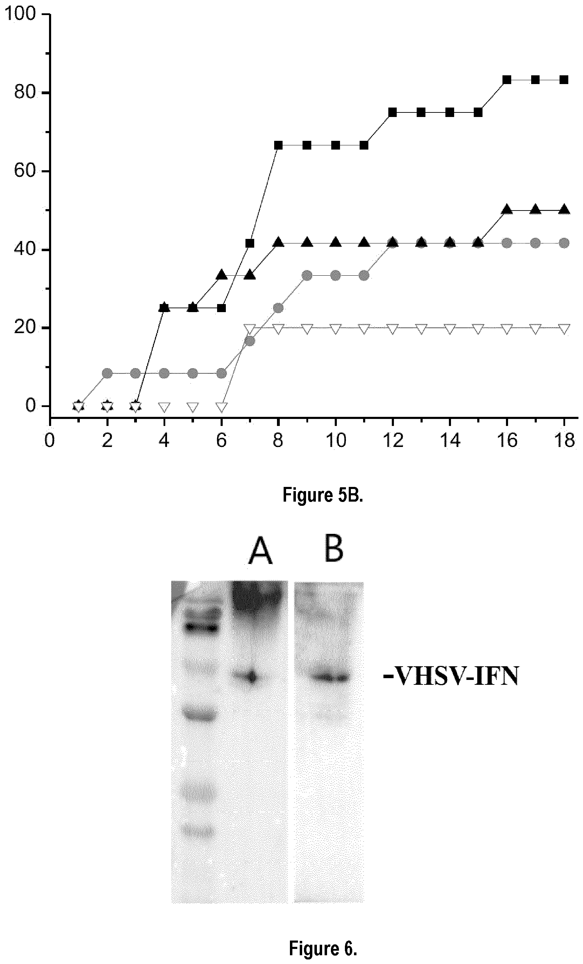

Figure 6. Detection of antigen in vaccinal feed. Western Blot with an anti-His-tag antibody to detect VHSV-IFN^{NP} after feed manufacturing by extrusion and final delipidation and NaOH extraction. A is the lane loaded with a total protein of 25 µg. B is the lane loaded with a total protein of 50 µg.

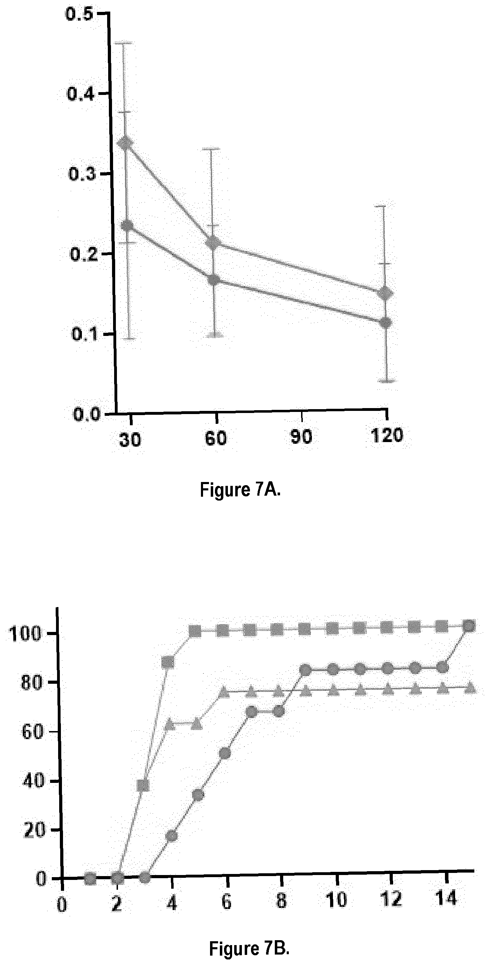

Figure 7. Levels of protection conferred by the NPs vaccinal feed.
   (A) Presence of anti-VHSV-G protein specific antibodies in sera from trout immunized with in-feed VHSV^{NP} (SEQ ID NO: 15) or VHSV-IFN^{NP} (SEQ ID NO: 16). Circles line refers to rainbow trout fed with iRFP^{NP}. Diamonds line refers to rainbow trout fed with VHSV-IFN^{NP}. Bars represent the average values and standard deviations. The x-axis represents the Inverse of sera dilution. The y-axis represents the absorbance at 450 nm.
      SEQ ID NO: 15 is 
TVTFCGTEWIKTDLGDLIQVTGPGGTRKLTPNKCVNTDIQMRGATDDFSYLNHLITNMAQRTECLDAHSDITAS
GKVSSFLLSKFRPSHPGPGKAHYLLDGQIMRGDCDYEAVVSINYNRAQYKTMNNTWKSWKRVDNNTDGYD
GMIFGDKLIIPDIEKYQSVYDSGMLVQRNLVEVPHLSIVFVSNTSDLSTNHIHTHHHHHH.
      SEQ ID NO: 16 is 
AQFTSINMKRNIDKLKVHYKISKDQLFNGNPVFPKDTFEDSEQRVLMSVVLDVYLSIFGQMLNQTGDQEMIESL
KYVKGKIQDLQKHYFLGRIPELRTHLQNLWAIETSDTTVQGKALSEFITIYEKASKLALKFHLKKDNRRKRRQAQ
RLKSHIMGGSSRSSTVTFCGTEWIKTDLGDLIQVTGPGGTRKLTPNKCVNTDIQMRGATDDFSYLNHLITNMA
QRTECLDAHSDITASGKVSSFLLSKFRPSHPGPGKAHYLLDGQIMRGDCDYEAVVSINYNRAQYKTMNNTWK
SWKRVDNNTDGYDGMIFGDKLIIPDIEKYQSVYDSGMLVQRNLVEVPHLSIVFVSNTSDLSTNHIHTHHHHHH.
   (B) Cumulative mortality of VHSV Infected rainbow trout after in-feed vaccination. Squares line refers to rainbow trout fed with iRFP^{NP}. Triangles line refers to rainbow trout fed with VHSV-IFN^{NP}. Fish injected with medium were used as the control (circles line). The x-axis refers to days post-challenge. The y-axis refers to the percentage of cumulative mortality.

### Detailed description of the invention

Unless defined otherwise, all technical and scientific terms used herein have the same meaning as is commonly understood by one of skill in the art to which this invention belongs at the time of filling. However, in the event of any latent ambiguity, definitions provided herein take precedent over any dictionary or extrinsic definition. Further, unless otherwise required by context, singular terms shall include pluralities and plural terms shall include the singular.

As used herein, the indefinite articles "a" and "an" are synonymous with "at least one" or "one or more." Unless indicated otherwise, definite articles used herein, such as "the" also include the plural of the noun.

A "polypeptide" is a chain of amino acids linked by a peptide bond (i.e., amide bonds) including from more than 10 amino acids. They are biological polymers and are called "proteins" when the number of amino acid residues is over 50 amino acids. As the skilled person will know, they can be obtained by synthetic routes, for example by solid-phase peptide synthesis on a rink amide resin using Fmoc-α-amine-protected amino acid. They can also be obtained from the recombinant gene technology, through genetic engineering of a fusion gene. This typically involves removing the stop codon from a cDNA sequence coding for the first protein, then appending the cDNA sequence of the second protein in frame through ligation or overlap extension PCR. That DNA sequence will then be expressed by a cell as a single protein. The protein can be engineered to include the full sequence of both original proteins, or only a portion of either. If the two entities are proteins, often linker (or "spacer") peptides are also added, which make it more likely that the proteins fold independently and behave as expected. Translation of this fusion gene results in a single or multiple polypeptides with functional properties derived from each of the original proteins. For "polypeptide" in this description is also to be understood a molecule of peptide/protein nature comprising two independent polypeptides coded for different or the same genes that are linked by a linker molecule other than a peptide. In this later scenario the compound of formula (I) is still considered a polypeptide that could also be referred as a conjugate of formula (I) or as a compound of formula (I).

A "polynucleotide" is to be understood as a nucleic acid molecule (DNA or RNA) comprising deoxyribonucleotides or ribonucleotides. Nucleic acid can be single or double stranded, and it includes, but it is not limited to, nucleotide sequences coding for polypeptides. In this description, polynucleotides used for the obtaining of the polypeptides by recombinant technology have a varying length depending on the construct. They have a length from at least 1000 nt nucleotides up to 4000 nt, and the mean of sizes of different constructs is 2000 nt.

When the polynucleotides are fusion genes, they are also known as "gene constructs" A "gene construct" according to the invention can also be named as an "expression cassette". It refers to a polynucleotide sequence including in turn a sequence coding for a protein of interest, which is operatively linked to an expression promoter, said promoter controlling expression of the sequence coding for the protein. For "operatively linked" is to be understood that the sequence coding for the protein is disposed after the sequence of the promoter (in the 5'-3' direction), or near the promoter in case restriction sites are included, or other stabilizing elements of the gene construction are present. The gene constructs (expression cassette) may also comprise small fragments with useful sequences to adapt it to more complex expression systems (vectors, plasmids), or a polyadenylation tail disposed after the sequence coding for the protein of interest. The expression cassette itself is also a expression system, being vectors or plasmids further used to protect the gene construct, or to promote entrance to cells in case of viral vectors. The "promoters" are of DNA regions that initiate transcription of a particular genes. Promoters are located near the transcription start sites of genes, on the same strand and upstream on the DNA (towards the 5' region of the sense strand). Promoters can be about 100-1000 base pairs long. A "constitutive promoter" is a promoter that is active in all circumstances in the cell, contrary to others that are regulated, becoming active in the cell only in response to specific stimuli, such as "inducible promoters". For the efficient expression of the polypeptide codified by the polynucleotide, the later is inserted in a vector, which is a DNA molecule used as a vehicle to artificially carry foreign genetic material into another cell, where it can be replicated and/or expressed (e.g., plasmid, cosmid, Lambda phages). A vector containing foreign DNA is termed recombinant DNA. The four major types of vectors are plasmids, viral vectors, cosmids, and artificial chromosomes. These vectors may carry elements (sequences) the skilled person will know aiding in the expression and stability of the expressed sequence, as well as tags or labels to purify the recombinant expressed polypeptide. The vectors are transfected to appropriate cell systems (host cells), which by means of their replicative and expression biochemical machinery will allow the obtaining of the recombinant polypeptide.

The term "nanopellet" (usually written as NanoPellet and abbreviated NP) or "nanoparticle" as used herein, refers generally to a particle with at least two dimensions at the nanoscale, particularly with all three dimensions at the nanoscale (1-1000 nm), although few micras are also to be considered within the nanoscale (1-1200 nm). As regards the shape (i.e., morphology) of the nanoparticles/nanopellets described herein, there are included particles with an ellipsoid shape, spheres. In a particular embodiment the nanoparticle is an ellipsoid. As used herein, the term "size" refers to a characteristic physical dimension. For example, in the case of a nanoparticle that is substantially spherical, the size of the nanoparticle corresponds to the diameter of the nanoparticle. When referring to a set of nanoparticles as being of a particular size, it is contemplated that the set of nanoparticles can have a distribution of sizes around the specified size. Thus, as used herein, a size of a set of nanoparticles can refer to a mode of a distribution of sizes, such as a peak size of the distribution of sizes. In addition, when not perfectly spherical, such as an ellipsoid, the diameter is the equivalent diameter of the spherical body including the object. When in this description the diameter or any physical dimension (i.e., width and length and high) of the nanoparticle/nanopellet is mentioned, it relates to dimension measured by Electron Microscopy, in particular Field Emission Scanning Electron Microscopy images (FESEM).

The term "food composition" or "food product" is used herein in its broadest meaning, including any type of product, in any form of presentation, which can be ingested by an animal, including humans. In particular it includes the common food that is used to feed aquatic animals, in particular fish or teleost and crustacean. An example of a fish diets are dry pellets made of 70% vegetable constituents and 30% marine raw matters such as fishmeal and fish oil. The fish feed contains vegetable components originating from plants such as soy, corn, rapeseed, sunflowers, broad beans, and wheat, which serve as proteins, carbohydrates, and fat sources. Other marine ingredients, such as fishmeal and fish oil are made from byproducts of the seafood processing industry that are not suitable for human consumption.

As used herein, the term "immunogenic" or "immunological composition" refers to material which elicits an immunological response in the host of a cellular or antibody-mediated immune response type to the composition upon administration to a vertebrate, including humans. The immunogenic composition comprises molecules with antigenic properties, such as immunogenic polypeptides. An immunogenic polypeptide is generally referred to as antigenic. A molecule is "antigenic" when it is capable of specifically interacting with an antigen recognition molecule of the immune system, such as an immunoglobulin (antibody) or T cell antigen receptor. An antigenic polypeptide contains an epitope of at least about five, and particularly at least about 10, at least 15, at least 20 or at least 50 amino acids. An antigenic portion of a polypeptide, also referred to as an epitope, can be that portion that is immunodominant for antibody or T cell receptor recognition, or it can be a portion used to generate an antibody to the molecule by conjugating the antigenic portion to a carrier polypeptide for immunization. The immunogenic composition relates according to this description, to the active molecule, composition comprising said molecule, or composition comprising more than one antigenic molecule to which a particular immune reaction is desired.

The term "antigen" refers to a molecule against which a subject can initiate an immune response, e.g., a humoral and/or cellular immune response. Depending on the intended function of the composition, one or more antigens may be included.

In the sense of the present disclosure, the term "fragment thereof" refers to fragment or fragments that maintain the activity of the polypeptide from which they derive, for example, fragments with interferon activity as in-vaccine adjuvant or fragments with immunogenicity of the viral antigen

As for the expression "immunologically effective amount," or "immunologically effective dose" means the administration of that amount or dose of antigen, either in a single dose or as part of a series, that elicits, or is able to elicit, an immune response that reduces the incidence of or lessens the severity of Infection or incident of disease in an animal for either the treatment or prevention of disease. The immunologically effective amount or effective dose is also able for inducing the production of antibody for either the treatment or prevention of disease. This amount will vary depending upon a variety of factors, including the physical condition of the subject, and can be readily determined by someone of skill in the art.

The term "medicament" as used herein is synonymous of a pharmaceutical or veterinary drug (also referred to as medicine, medication, or simply drug) use to cure, treat or prevent disease in animals, including humans, as widely accepted. Drugs are classified in various ways. One key distinction is between traditional small-molecule drugs, usually derived from chemical synthesis, and biopharmaceuticals, which include recombinant proteins, vaccines, blood products used therapeutically (such as Intravenous Immunoglobulin (IVIG)), gene therapy, monoclonal antibodies and cell therapy (for instance, stem-cell therapies). In the present invention medicament preferably is a veterinary medicament, and even more preferably is a vaccine for veterinary use.

The term "vaccine" as used herein, means an immunogenic composition of the invention accompanied by adequate excipients and/or carriers, that when administered to an animal, elicits, or is able to elicit, directly or indirectly, an immune response in the animal. Particularly, the vaccines of the present invention elicit an immunological response in the host of a cellular or antibody-mediated type upon administration to the subject that it is protective. The term "oral vaccine" means that the vaccine is in particular appropriated for oral administration, thus including the galenic for being stable and to deliver the immunogenic composition at the desired place and in an adequate conformation to induce the immunological reaction.

The term "carrier" is to be understood as a pharmaceutically acceptable component other than the immunogenic component. The carrier can be organic, inorganic, or both. Suitable carriers well known to those of skill in the art and include, without limitation, large, slowly metabolized macromolecules such as proteins, polysaccharides, polylactic acids, polyglycolic acids, polymeric amino acids, amino acid copolymers, lipid aggregates (such as oil droplets or liposomes) and inactive virus particles.

The expression "pharmaceutically acceptable excipients or carriers" refers to pharmaceutically acceptable materials, compositions or vehicles. Each component must be pharmaceutically acceptable in the sense of being compatible with the other ingredients of the pharmaceutical composition. It must also be suitable for use in contact with the tissue or organ of humans and animals without excessive toxicity, irritation, allergic response, immunogenicity or other problems or complications commensurate with a reasonable benefit/risk ratio.

As above exposed, the inventors propose for the first time a fusion polypeptide comprising at least a viral antigenic module and an interferon module, and an immunogenic composition and/or vaccine comprising said fusion polypeptide, for use as a medicament in fish or teleost and certain crustaceans.

All particular embodiments for the fusion polypeptide for use as a medicament disclosed herewith apply also to the immunogenic composition and/or the vaccine for use as a medicament comprising said fusion polypeptide.

As previously indicated, a first aspect of the invention is a polypeptide comprising the amino acid sequence of an interferon or a fragment thereof, the amino acid sequence of a viral antigen or a fragment thereof and, optionally, a linker between them.

In a particular embodiment of the first aspect the polypeptide has formula (I):

R1-(L)n-R2

wherein:
n is an integer selected from 0 or 1;
R1, which is at the N-terminal end of the polypeptide, comprises the amino acid sequence of an interferon or a fragment thereof;
L is a linker; and
R2, which is at the C-terminal end of the polypeptide, comprises the amino acid sequence of a viral antigen or a fragment thereof.

In other words, the polypeptide can be defined as having formula (I)

R1-(L)n-R2

wherein:
n is an integer selected from 0 or 1;
R1, which is at the N-terminal end of the polypeptide, is an in-vaccine adjuvant;
L is a peptide linker; and
R2, which is at the C-terminal end of the polypeptide, is an antigenic protein module.

Thus, this polypeptide of formula (I) is, in certain embodiments, a fusion protein.

In a particular embodiment of the first aspect, the polypeptide is one in which R2 comprises the amino acid sequence of a viral antigen of a virus causing disease in aquatic animals selected from fish or teleost and crustacean. More in particular, fish or teleost and crustacean selected from the group consisting of Salmonids, turbot, carp, cyprinids, seabass, common sole, turbot, barramundi, Seabream and prawns.

In another particular embodiment, the polypeptide is one in which R2 comprises the amino acid sequence of a viral antigen of a virus causing disease. In particular, said virus selected from the group of Infectious Pancreatic Necrosis Virus (IPNV), Viral Hemorrhagic Septicemia Virus (VHSV), Viral Nervous Necrosis Virus (VNNV) or its specific serotype Red-spotted Grouper Nervous Necrosis Virus/Striped Jack Nervous Necrosis Virus (RGNNV/SJNNV), Lymphocystis Disease Virus (LCDV) and Spring Viraemia of Carp Virus (SVCV).

In particular, said viral antigen is a viral protein or a fragment thereof selected from the group of glycoproteins, capsid proteins, major capsid proteins or fragments thereof. Thus, it is a protein or fragment capable of causing immune reaction.

In a more particular embodiment, the virus causing disease is Spring Viremia Carp Virus. And in particular, the antigen module of said virus contained in the polypeptide of the first aspect is the G3 region of the Glycoprotein G of said virus.

In another particular embodiment, of the first aspect the polypeptide is one in which R2 comprises SEQ ID NO: 1 or a sequence at least 85%, or at least 90%, or at least 95%, or at least 96%, or at least 97%, or at least 98%, or at least 99% identical to SEQ ID NO: 1.

In another particular embodiment of the first aspect, the polypeptide is one in which R2 consists of SEQ ID NO: 1.

In a more particular embodiment, the virus causing disease is Viral Hemorrhagic Septicemia Virus. And in particular, the antigen module of said virus contained in the polypeptide of the first aspect is the fragment 16 of the Glycoprotein G of said virus.

In another particular embodiment, of the first aspect the polypeptide is one in which R2 comprises SEQ ID NO: 5 or a sequence at least 85%, or at least 90%, or at least 95%, or at least 96%, or at least 97%, or at least 98%, or at least 99% identical to SEQ ID NO: 5.

In another particular embodiment of the first aspect, the polypeptide is one in which R2 consists of SEQ ID NO: 5.

In a more particular embodiment, the virus causing disease is Viral Nervous Necrosis Virus. And in particular, the antigen module of said virus contained in the polypeptide of the first aspect is a fragment of the coat protein gene of said virus. In a more particular embodiment the coat protein gene is from the Iberian betanodavirus isolate (strain SpSs-IAuscl60.03), which is a reassortant RGNNV/SJNNV strain.

In another particular embodiment, of the first aspect the polypeptide is one in which R2 comprises SEQ ID NO: 6 or a sequence at least 85%, or at least 90%, or at least 95%, or at least 96%, or at least 97%, or at least 98%, or at least 99% identical to SEQ ID NO: 6.

In another particular embodiment of the first aspect, the polypeptide is one in which R2 consists in SEQ ID NO: 6.

The amino acid sequences of viral antigens of particular embodiments of all the aspects of the invention are given in Table 1:

| Description of the sequence (origin) | SEQ ID No. | Amino acid sequence |
|---|---|---|
| G3 region, AA 254-381, which is the fragment of glycoprotein G of SVCV Uniprot KB accession number Q91DS0, version 1 of the sequence of December, 1, 2001, and version 76 of the database release. | 1 | VEKTAGTLTTIHDNVPKCVDGTLVSGHR PGLDLIDTVFNLENVVEYTLCEGTKRKIN KQEKLTSVDLSYLAPRIGGFGSVFRVRN GTLERGSTTYIRIEVEGPIVDSLNGTDPR TNASRVFWDDWELD |
| IPNV capsid protein 2 from the IPNV (strain Sp 31-75), Chain (PRO_0000227873) position 1-442. Uniprot KB accession number Q703G9, version 1 of the sequence of July 5, 2004, and version 76 of the database release. | 4 | MNTNKATATYLKSIMLPETGPASIPDDIT ERHILKQETSSYNLEVSESGSGVLVCFP GAPGSRIGAHYRWNANQTGLEFDQWL ETSQDLK KAFNYGRLISRKYDIQSSTLP AGLYALNGTLNAATFEGSLSEVESLTYN SLMSLTTNPQDKVNNQLVTKGVTVLNL PTGFDKPYVRLEDETPQGLQSMNGAK MRCTAAIAPRRYEIDLPSQRLPPVPATG TLTTLYEGNADIVNSTTVTGDINFSLAEQ PADETKFDFQLDFMGLDNDVPVVTVVS SVLATNDNYRGVSAKMTQSIPTENITKPI TRVKLSYKINQQTAIGNVATLGTMGPAS VSFSSGNGNVPGVLRPITLVAYEKMTPL SILTVAGVSNYELIPNPELLKNMVTRYG KYDPEGLNYAKMILSHREELDIRTVWRT EEYKERTRVFNEITDFSSDLPTSKA |
| VHSV glycoprotein G fragment 16 (amino acid residues 252-449) of the VHSV (07-71) G protein sequence, UniProt KB accession number P27662, version 1 of the sequence of August 1, 1992, and version 81 of the database release. | 5 | TVTFCGTEWIKTDLGDLIQVTGPGGTRK LTPNKCVNTDIQMRGATDDFSYLNHLIT NMAQRTECLDAHSDITASGKVSSFLLSK FRPSHPGPGKAHYLLDGQIMRGDCDYE AVVSINYNRAQYKTMNNTWKSWKRVD NNTDGYDGMIFGDKLIIPDIEKYQSVYD SGMLVQRNLVEVPHLSIVFVSNTSDLST NHIHT |
| VNNV coat protein gene from the Iberian betanodavirus isolate (strain SpSs-IAuscl60.03), which is a reassortant RGNNV/SJNNV strain. UniProt KB accession number D0QU14, version 2 of November 29, 2014, and version 26 of the database release. | 6 | MVRKGDKKLAKPATTKAANPQPRRRAT QRRRSGRADAPLAKASTITGFGRATND VHISGMSRIAQAVVPAGTGTDGKIIVDS TIVPELLPRLGHAARIFQRYAVETLEFEI QPMCPANTGGGYVAGFLPNPTDNDHT FDALQATRGAVVAKWWESRTVRPQYT RTLLWTSTGKEQRLTSPGRLVLLCVGS NTDVVNVSVMCRWSVRLSVPSLETPED TTAPITTQAPLHNDSINNGYTGFRSILLG STQLDLAPANAVFVTDKPLPIDYSLGVG DVDRAVYWHLRKKAGDTQVPAGYFDW GLWDDFNKTFTVGAPYYSDQQPRQILL PAGTLFTRVDSEN |

In another particular embodiment of the first aspect, the polypeptide is one in which R1 comprises the amino acid sequence of an interferon or a fragment thereof of an animal species selected from fish or teleost and crustacean, the interferon selected from the group consisting of interferons Type I and Type II.

Particularly, the interferon or a fragment thereof is of fish and/or crustacean selected from the group consisting of Salmonids, turbot, carp, cyprinids, seabass, common sole, turbot, barramundi, Seabream and/or prawns.

And in a particular embodiment, the interferon or a fragment thereof from the above embodiment is selected from the Type II interferon group.

In a more particular embodiment, the polypeptide is one in which R1 comprises the amino acid sequence or a fragment thereof of the interferon γ (gamma).

In a more particular embodiment, the interferon or fragment thereof is selected from the group of Trout interferon γ, Turbot interferon γ, Seabream interferon, Seabass interferon γ, zebra fish and/or carp interferon Y.

Yet in a more particular embodiment, the interferon γ or a fragment thereof is the zebrafish or carp interferon Y.

In another particular embodiment, the polypeptide is one in which R1 comprises SEQ ID NO: 2 or a sequence at least 85%, or at least 90%, or at least 95%, or at least 96%, or at least 97%, or at least 98%, or at least 99% identical to SEQ ID NO: 2.

In another particular embodiment of the first aspect, the polypeptide is one in which R1 consists in SEQ ID NO: 2.

The amino acid sequences of interferon or fragments thereof of all the aspects of the invention are given in Table 2:

| Description of the sequence (origin) | SEQ ID No. | Amino acid sequence |
|---|---|---|
| IFN gamma 1 from D. *rerio* (27-175). Uniprot KB accession number Q75S22, version 1 of the sequence of July 5, 2004, and version 116 of the database release. | 2 | SVPENLDKSIEELKAYYIKEDSQLHN AHPIFLRILKDLKVNLEESEQNLLMSI VMDTYSRIFTRMQNDSVDEATKERL AHVQEHLKKLQESYFPGKSAELRTY AETLWAIKENDPIVQRKALFELKRVY REATLLKNLKNKERKRRQAK |
| Trout IFNγ (25-180). Uniprot KB accession number Q5QSL2_ONCMY, version 1 of the sequence of January 4, 2005, and version 70 of the database release. | 7 | AQFTSINMKRNIDKLKVHYKISKDQL FNGNPVFPKDTFEDSEQRVLMSVV LDVYLSIFGQMLNQTGDQEMIESLK YVKGKIQDLQKHYFLGRIPELRTHLQ NLWAIETSDTTVQGKALSEFITIYEK ASKLALKFHLKKDNRRKRRQAQRLK SHIM |
| Scophthalmus maximus IFNγ without signal peptide (23-180). Uniprot KB accession number A0A1 J0PK52, version 1 of the sequence of February 15, 2017, and version 16 of the database release. | 8 | SHVSARMNRTIQNLLLHYKIPPKDRF NGKPVFSREPLAGKVETKKLFMVG VLETYEKLIGQMLTHLPTPNPPTTGI HVRLGAATAAATAPGAGGEVGKEL NFILKKVQELRKHRYQEQSELLQGL EALGKIQMDNFVVQSKALWELPWL YEEASSISN |

In a particular embodiment, the IFN gamma 1 from D. *rerio* (27-175) as defined in Table 2 is also useful for other cyprinids species. In a particular embodiment, for *Cyprinus carpio.*

In a particular embodiment, the trout IFNγ (25-180) as defined in Table 2, is also useful for other salmonid species. In a particular embodiment, for *Salmo salar.*

In another particular embodiment, the polypeptide is one in which "n" is 1, and the linker L is selected from a peptide with a length from 2 to 15 amino acids.

In a particular embodiment, the peptide length is any selected from 2, 3, 4, 5, 6, 7, 8, 9, 10, 11, 12, 13, 14, 15.

In another particular embodiment, the polypeptide is one in which the linker L is a peptide comprising or consisting in SEQ ID NO: 3.

The amino acid sequence of the linker is given in Table 3:

| SEQ ID No. | Amino acid sequence (in N-C-terminal direction) |
|---|---|
| 3 | GGSSRSS |

Linkers of different nature would also work as long as the spatial distribution of the linker set forth in SEQ ID No. 3 is maintained.

In another particular embodiment, the linker L is selected from the group of consisting of the above-indicated peptides, PEGs, other polymeric compounds (such as nucleic acids, polylactide acid, polycaprolactones, polyvynilsuccinates, polyhidroxybutirates). Even in the case the linker (L) is not of peptide nature, the compound of formula (I) is still considered a polypeptide according to this description, although in such a case it could also be referred as a conjugate of formula (I).

Independently of the nature of the linker (L), when present in formula (I), it allows the spatial disposition that makes R1 and R2 more visible to the immune system (i.e., spatially disposed to allow a response of the immune system). Alternatively, when the linker L is absent (n=0), the protein nature of R1 and R2 also allows the activation of the immune system when administered to an animal.

As mentioned above the invention provides a polynucleotide which encodes the compound as defined in the first aspect. In a particular embodiment of the polynucleotide, optionally in combination with any of the embodiments provided above and below, the polynucleotide is DNA or RNA.

As mentioned before, the invention also provides a vector comprising the polynucleotide as disclosed above. Examples of suitable vectors include those conventionally used in biomedicine and known to the skilled person.

As mentioned above, the invention also provides a cell system which is transformed or transfected with the polynucleotide or the vector of the invention. More precisely, the invention provides a host cell which is transformed or transfected with the polynucleotide and/or the vector as defined above, and it also provides a cell culture comprising said host cell. The skilled person would know which host cells are suitable for the synthesis of the protein of the invention.

As mentioned above, in a second aspect, the invention provides a protein nanopellet (i.e., nanoparticle) comprising one or more of the polypeptides defined in the first aspect and with a particle diameter from 400 nm to 1500 nm, measured by electron microscopy.

In a more particular embodiment, the nanopellet is spheroid, and it has a width from 250 nm to 1000 nm, and a length from 500 nm to 1500 nm. The measures of these dimensions obtained, as indicated by EM, in particular by Field emission scanning electron microscopy (FESEM).

In a more particular embodiment, the nanopellet has a width from 500 nm to 750 nm and a length from 750 nm to 1200 nm.

In a more particular embodiment, the nanopellet has a width from 519,1 nm to 728,9 nm and a length from 765,1 nm to 1198,9 nm.

In a more particular embodiment, the protein nanopellet comprises one or more of endotoxin, nucleic acids or peptidoglycans.

The obtention of the nanopellets is carried out by means of the current recombinant technology and protein expression techniques known by the skilled person in the art.

In a particular embodiment, the protein nanopellet is obtainable by a process comprising the following steps:
- Transformation of a host cell with plasmids or vectors comprising a polynucleotide encoding a polypeptide as described in the first aspect, or in any of its embodiments, to obtain a host transformed cell;
- Culture of the host transformed cell in an appropriated culture media;
- Induction of expression of the polypeptide encoded in the polynucleotide, to obtain such a polypeptide in form of nanopellets; and
- Purification of the polypeptide nanopellets from the cell host culture media.

Thus, it is also herewith disclosed a method for the obtention of the nanopellets of the invention, the method comprising the previous listed steps. All the particular embodiments of the process are equally applicable to the nanopellets obtainable by means of the same.

In a particular embodiment of the process, the host cell (i.e., cell system for the production of the nanopellets) is a bacterial cell.

In a more particular embodiment, the cell system for the production of the nanopellets is *E*. *coli* bacteria. More in particular it is strain BL21 of *E*. *coli.*

In another particular embodiment of the process, the step of purification is carried out by enzymatic and/or mechanical disruption of the cell host, optionally in presence of a one or more of solvents, detergents, and surfactants, in particular non-ionic surfactants, and protease inhibitors.

The skilled person in the art will know which, enzymes, reagents and/or physical means are currently employed for the purification of a compound of interest (i.e., a protein pellet or inclusion body) from a transformed cell culture.

Examples of enzymes include the lysozyme. Examples of physical or mechanical means include centrifugation steps, frozen and thaw cycles.

Examples of non-ionic surfactants include Triton X-100.

Other non-ionic surfactants are food grade non-ionic surfactants, in particular selected from polyglycerol alkyl ethers, glucosyl dialkyl ethers, crownethers, ester-linked surfactants, polyoxyethylene alkyl ethers, Brij, Spans (sorbitan esters) and Tweens (Polysorbates). Food grade means that are compounds useful in the processes for obtaining food without any danger for the final consumers.

In even a more particular embodiment the process for obtaining the nanopellets of the invention comprises the following steps:
- Transformation of a host cell, in particular *E.coli,* with plasmids or vectors comprising a polynucleotide encoding a polypeptide as described in the first aspect, or in any of its embodiments, to obtain a host transformed cell;
- Culture of the host cell in LB medium supplemented with an antibiotic and incubated at a temperature optimal for the host cells, in particular at 37°C, with agitation in particular at 250 rpm until reaching and OD550 of 0.6;
- Induction of expression of the polypeptide encoded in the polynucleotide, to obtain such the polypeptide in form of nanopellets, by addition of an inductor, in particular the isopropyl β-D-thiogalactoside (IPTG), for a predetermined period of time, in particular from 3 to 6 h; and
- Purification of the polypeptide from the cell host culture media through the particular combination of enzymatic and mechanical disruption, said of enzymatic and mechanical disruption comprising: 1) digestion with lysozyme (1 µg/ml) and phenylmethylsulfonyl fluoride (PMSF) (0.4 mM) added to the culture and incubation for 2h-3h at 37°C, under agitation at 250 rpm; 2) Frozen and thawing the cells in the presence of a non-ionic surfactant and incubation for 1 h-2 h under agitation at room temperature (RT); 3) harvesting of the nanopellets by centrifugation and resuspended in Tris buffer; 4) DNAse treatment, for 1 h-2 h at 37°C under agitation; 5) Optional freeze/thaw cycles to separate any cell and/or cell debris; 6) Centrifugation, in particular at 15000 x g 15 min, to pelletize the nanopellets , which are optionally stored until use (in particular at low temperatures, such as at -80°C).

As mentioned above, a third aspect of the invention is an immunogenic composition comprising one or more polypeptides as defined in the first aspect, and/or the protein nanopellet as defined in the second aspect.

As mentioned above, a fourth aspect of the invention is a vaccine comprising the immunogenic composition as defined above, and a pharmaceutically acceptable excipient and/or carrier. Thus, the vaccine comprises an immunogenically effective amount of the immunogenic composition.

In a particular embodiment, the vaccine further comprises an adjuvant.

As mentioned above, the fifth aspect of the invention is a polypeptide as defined in the first aspect and/or the protein nanopellet as defined in the second aspect, and/or the immunogenic composition as defined in the third aspect, and/or the vaccine as defined in the fourth aspect, for use in therapy, that is, as a medicament.

In a particular embodiment, the polypeptide and/or the protein nanopellet, and/or the immunogenic composition, and/or the vaccine as defined above, is for use in the prevention and/or treatment of a viral disease in fish or teleost and crustaceans.

This embodiment can also be formulated as the use of the polypeptide and/or the protein nanopellet, and/or the immunogenic composition, and/or the vaccine as defined above for the manufacture of a medicament, for the treatment and/or prevention of Infections or diseases caused by viral diseases in fish or crustacean. This embodiment can also be formulated as a method of immunizing fish or teleost and crustacean in need thereof with an immunologically effective amount of a polypeptide and/or a protein nanopellet, and/or an immunogenic composition, and/or a vaccine as defined above, in particular for treating and/or preventing Infections or disease caused by viruses.

In a more particular embodiment, the Infections or disease are caused by one or more viruses selected from the group consisting of pancreatic necrosis virus (IPNV), Viral Hemorrhagic Septicemia Virus (VHSV), Viral Nervous Necrosis Virus (VNNV) and Spring Viraemia of Carp Virus (SVCV). More in particular, the Infections or disease are caused by SVCV.

Particularly, the fish or crustacean is selected from the group of Salmonids, turbot, carp, cyprinids, seabass, common sole, turbot, barramundi, Seabream and/or prawns.

More particularly, the polypeptide and/or the protein nanopellet, and/or the immunogenic composition, and/or the vaccine is for use in preventing and/or treating SVCV in carp or zebrafish.

As pointed above, oral administration is highly desirable for therapy and prophylaxis for fish or teleost and crustacean viral diseases, particularly for species with moderate market value and high production. The present invention is a biocompatible, cheap and modular platform that allow the production of recombinant proteins in a highly stable conformation.

Thus, in a particular embodiment of the invention, the polypeptide as defined in the first aspect and/or the protein nanopellet as defined in the sixth aspect, and/or the vaccine as defined in the seventh aspect, for use in therapy is orally administered.

Another particular embodiment of the invention is a vaccination kit of parts comprising:
(a) an immunogenic composition as defined in the third aspect;
(b) a pharmaceutically acceptable excipient and/or carrier;
(c) optionally an adjuvant; and
(d) optionally instructions for its use.

As mentioned above, the sixth aspect of the invention is a food composition comprising a polypeptide, and/or a protein nanopellet, and/or the immunogenic composition, and/or the vaccine, as defined above. The food composition comprises at least one edible ingredient.

In a particular embodiment of the sixth aspect the at least one edible ingredient is selected from the group of vegetable components, soy, corn, rapeseed, sunflowers, broad beans, wheat, marine ingredients, fishmeal and fish oil.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

### Examples

### Example 1. Design, Production, and Characterization of Nanostructured Viral Antigenic Proteins

### Viral Strains and Plasmids

Recombinant SVCV G protein was designed based on the glycoprotein G sequence (G3 region, AA 254-381, Uniprot acc. no: Q91DS0 as set forth in SEQ ID NO: 1). Recombinant SVCV G antigen with the interferon gamma module (SVCV-IFN) was based on the sequence of the G3 region (254-381. SEQ ID NO: 1) linked to the sequence of the IFN gamma 1 from *D*. *rerio* (27-175, SEQ ID NO: 2) (NCBI GenBank NP_998029.1). Clones were designed using the ORF and pET22b removing the periplasmic location signal and including a C terminal polyHis-tag. Clones were codon optimized for expression in *E. coli,* synthesized by GeneScript and subcloned into pET22b. Plasmids were transformed into *E. coli* BL21 (DE3) (Novagen). The red fluorescent protein (RFP), iRFP-His cloned into pET22b (Genscript), was used as a non-relevant control protein.

### Production of NPs, Purification, Quantification, and Fluorescent Labeling

Methods for recombinant protein production and characterization were previously reported (Thwaite *et al*., 2018). Briefly, some modifications were introduced for these particular constructs: *E. coli* were cultured overnight (O/N) in LB medium (Gibco) with ampicillin (100 µg/ml, Sigma-Aldrich) and recombinant protein expression in *E. coli* was induced with 1 mM Isopropyl β-D-1-thiogalactopyranoside (IPTG, Panreac) when OD550 nm reached 0.5-0.8. Growth was carried out for 4,5 h at 37°C. For NPs purification, the cell cultures were subjected to an enzymatic and mechanical disruption process (Torrealba, D., Parra, D., Seras-Franzoso, J., Vallejos-Vidal, E., Yero, D., Gibert, I., Villaverde, A., Garcia-Fruitós, E., Roher, N., 2016a. Nanostructured recombinant cytokines: A highly stable alternative to short-lived prophylactics. Biomaterials 107, 102-114. https://doi.org/10.1016/i.biomaterials.2016.08.043). Lysozyme (1,5 µg/ml, Roche), protease inhibitor complete EDTA-free Tablets (Roche) and phenyl-methanesulfonyl fluoride (PMSF, Roche) (0.4 mM) were added and incubated at 37°C for 2 h at 250 rpm. Cultures were then frozen at -80°C overnight (O/N). After thawing, Triton X-100 (Roche) was added (0.2% v/v) and stirred for 1 h at room temperature (RT). Then, DNase I (1 µg/ml) and MgSO4 (1 M) were added, and the cultures were incubated for 1 h at 37°C with shaking. NPs were harvested by centrifugation at 15000 x g, 15 min at 4°C and resuspended in lysis buffer (50 mM Tris HCl pH 8, 100 mM NaCl, 1 mM EDTA) at 1/10 the original culture volume. Finally, several freeze thaw cycles were performed to remove any remaining viable bacteria. Pellets of purified NPs, named SVCV^{NP} (SEQ ID NO: 13) and SVCV-IFN^{NP} (SEQ ID NO: 14) were stored at -80°C until use. Protein was semi-quantified by western blot using an anti-His-tag antibody (Genscript A00186-100), and the protein concentration was calculated using Image Lab 6.0.1 software (Bio-Rad). For flow cytometry or confocal microscopy, NPs were conjugated with Atto-488 NHS ester (Sigma-Aldrich) according to the manufacturer's instructions. Labeling efficiency was determined with the Nanodrop ND-1000 (Thermo Scientific).
SEQ ID NO: 13 is 
VEKTAGTLTTIHDNVPKCVDGTLVSGHRPGLDLIDTVFNLENVVEYTLCEGTKRKINKQEKLTSVDLSYLAPRIG
GFGSVFRVRNGTLERGSTTYIRIEVEGPIVDSLNGTDPRTNASRVFWDDWELDHHHHHH.
SEQ ID NO: 14 is

SVPENLDKSIEELKAYYIKEDSQLHNAHPIFLRILKDLKVNLEESEQNLLMSIVMDTYSRIFTRMQNDSVDEATK
ERLAHVQEHLKKLQESYFPGKSAELRTYAETLWAIKENDPIVQRKALFELKRVYREATLLKNLKNKERKRRQA
KGGSSRSSVEKTAGTLTTIHDNVPKCVDGTLVSGHRPGLDLIDTVFNLENVVEYTLCEGTKRKINKQEKLTSVD
LSYLAPRIGGFGSVFRVRNGTLERGSTTYIRIEVEGPIVDSLNGTDPRTNASRVFWDDWELDHHHHHH.

### Characterization of Viral Recombinant Protein NPs

Electron Microscopy was used to determine the external morphology and physical dimensions of the NPs. Samples were prepared by resuspending NPs at 100 µg/ml in mQ water, pipetting 20 µl onto silicon chips, and air-dried O/N. NPs were exposed to different in vitro conditions. For pH, NPs were incubated under gentle stirring at pH 2 and 10 for 3 h together with a control sample. For temperature, NPs were incubated for 5 min at 100 °C. After incubation all NPs were centrifuged at 10.000 xg for 5 min at 4 °C and resuspended at 10 and 50 µg/ml in phosphate buffered saline (PBS, Sigma-Aldrich) and finally 20 µl were deposited onto silicon chips, and air-dried O/N. Electron microscopy images were taken with a FESEM (Zeiss Merlin) and analyzed using Fiji package (National Institute of Health, USA). At least 60 particles were measured, and size distribution histograms were generated using Past 4.04 software (University of Oslo).

### Results

Two different recombinant viral proteins were produced in *E. coli* as bacterial IBs (i.e., NPs) with moderate to good yields: SVCV^{NP} 0.3 mg/l and SVCV-IFN^{NP} 3.6 mg/l. Expression of recombinant proteins is an empiric process with several experimental constraints that affect the production yield.

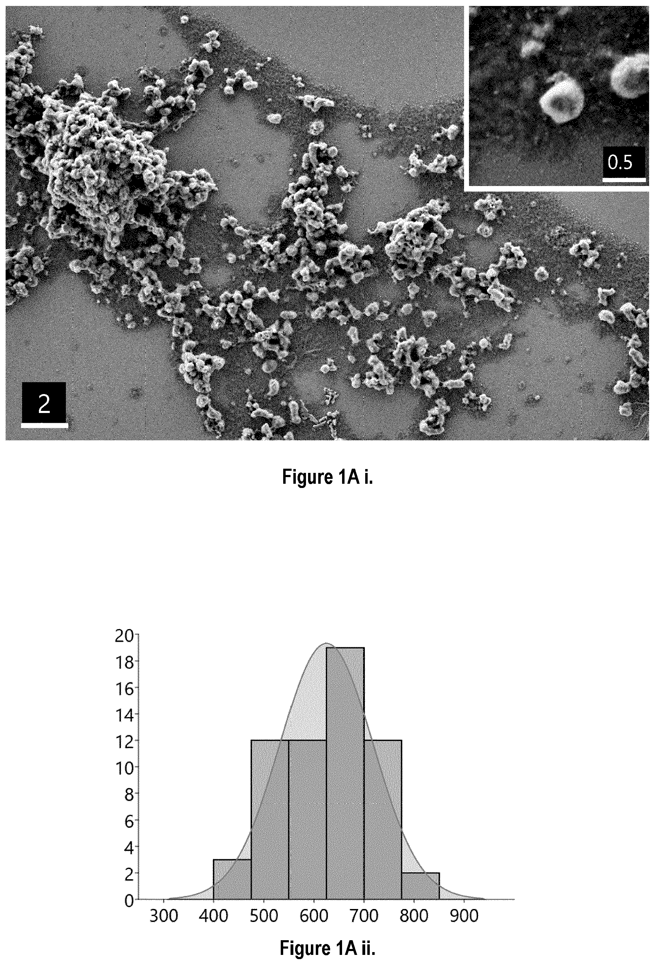

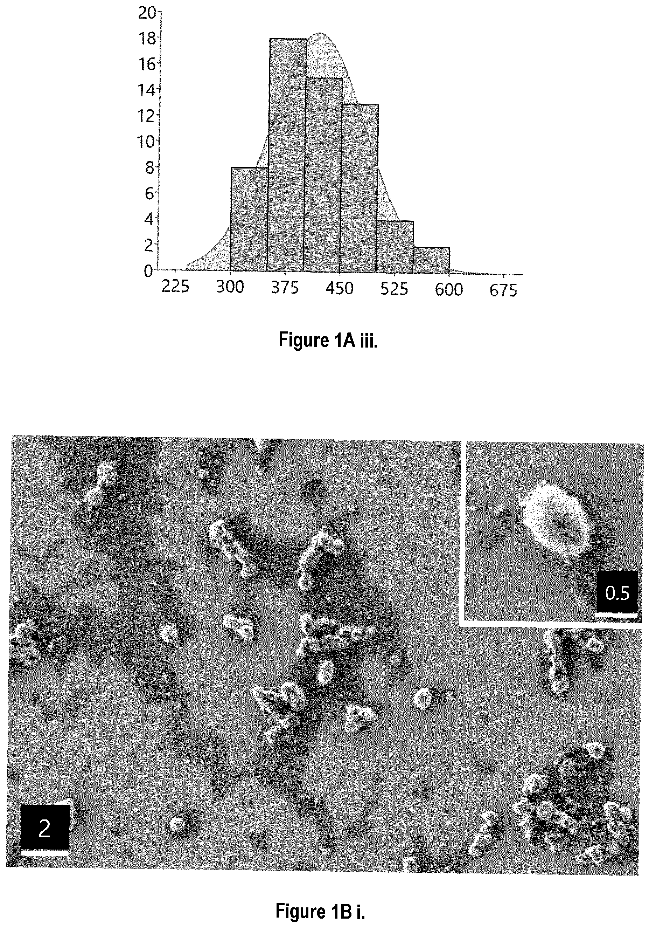

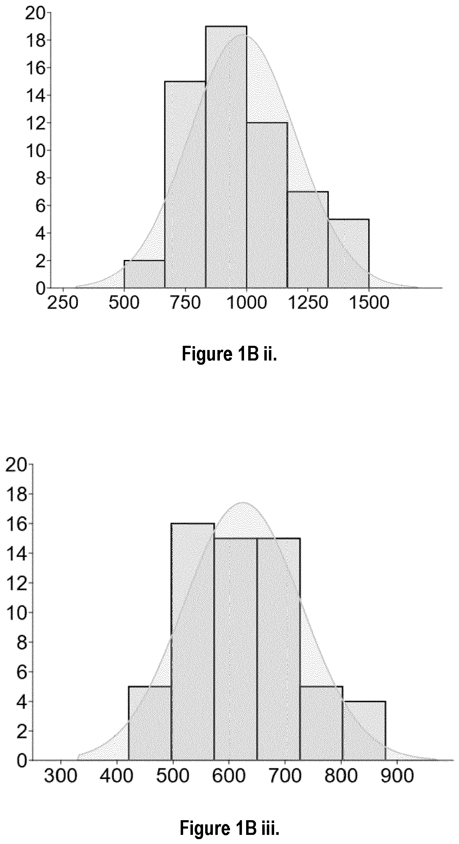

The size and morphology of both NPs were evaluated by FESEM (Figure 1Ai and Figure 1Bi). SVCV-IFN^{NP} is generally oviform (elliptical shaped) while SVCV^{NP} is rounder and smaller. Similar morphologies had been observed in other IBs produced in *E. coli* using the same strain BL21 (DE3) and in M15(pREP4) (Thwaite et *al.,* 2018). The size range is shown in Figures 1Aii, 1Aiii, 1Bii and 1Biii with average width and length being 420 ± 67.5 nm and 693 ± 154 nm for SVCV^{NP} and 624 ± 104.9 nm and 982 ± 216.9 nm for SVCV-IFN^{NP}, respectively. Morphological and structural integrity of NPs were evaluated under different environmental conditions of temperature and pH to assess the resistance of the NPs under industrial extrusion procedures and gastrointestinal pH conditions. SVCV^{NP} and SVCV-IFN^{NP} were subjected to high temperature (100°C) and extreme pH conditions (pH 2 and 10) and significant changes in size or morphology were not observed (data not shown).

### Example 2. Uptake of modular nanostructured antigens by ZFL cells

### Methods

### Cell culture and virus

Zebrafish ZFL cells (CRL-2643, ATCC) were cultured according to Thwaite et al. (Thwaite *et al.,* 2018) at 28°C and 5% CO2 in DMEM + GlutaMAX (Gibco), 10% heat-inactivated fetal bovine serum (FBS) (Gibco), 0.01 mg/ml insulin (Sigma-Aldrich), 50 ng/ml epidermal growth factor (Sigma-Aldrich), 2% (v/v) antibiotic/antimycotic (Gibco), and 0.5% (v/v) trout serum which had been filtered (0.20 µm filter Corning) and heat inactivated for 30 min at 56°C.

Zebrafish embryonic fibroblast (ZF4 cells) were cultivated at 28°C and 5 % CO₂ in RPMI medium (Gibco) supplemented with 10% FBS (Cultek), 1 mM pyruvate (Gibco), 2 mM L-glutamine (Gibco), 50 µg/ml gentamicin (Gibco) and 2 µg/ml amphotericin B (Gibco).

Fathead minnow (*Pimephales promelas*) EPC cells were maintained at 28°C and 5 % CO₂ in medium RPMI-1640 supplemented with 10% FBS, 1 mM pyruvate, 2 mM L-glutamine, 50 µg/ml gentamicin and 2 µg/mi amphotericin B.

SVCV (strain 56/70) originally isolated from carp (Fijan, N., Petrinec, Z., Sulimanovic, D., & Zwillenberg, L.O. (1971). Isolation of the viral causative agent from the acute form of Infectious dropsy of carp. Veterinarski Arhiv, 41, 125-138) was propagated in EPC cells at 28°C until a cytopathic effect was observed. Then, the cell culture medium was harvested, centrifuged at 4000 x g for 30 min, and virus aliquots were stored at - 80°C.

### Uptake of Nanostructured Viral Antigens by ZFL cells

To test cellular uptake, fluorescently labelled NPs were added to ZFL cells cultures at 70-80% confluence after 2-3 h incubation in minimal media (0-0.5% FBS) at the doses and times indicated below. For dose-response assays, SVCV^{NP} and SVCV-IFN^{NP} were added at 2.5, 5, 10, 20 and 40 µg/ml and cultures were then incubated O/N (12-14 h). For time-course assays, SVCV NPs were added at 5 and 10 µg/ml and cultures were simultaneously incubated for 12 and 24 h. For comparative assays, SVCV^{NP} and SVCV-IFN^{NP} were added at 5 and 10 µg/ml and cultures were then incubated O/N (12-14 h). All experiments were performed in duplicate. Post treatment, ZFL cells were washed in PBS and incubated at 28°C and 5% CO2 with 1 mg/ml Trypsin (Gibco) for 15 min. This strong trypsinization step aimed to remove NPs attached to the cell surface (Seras-Franzoso, J., Sánchez-Chardi, A., Garcia-Fruitós, E., Vázquez, E., Villaverde, A., 2016. Cellular uptake and intracellular fate of protein releasing bacterial amyloids in mammalian cells. Soft Matter 12, 3451-3460. https://doi.ora/10.1039/c5sm02930a). Then, two volumes of complete medium were added, and cells were pelleted by centrifugation at 300 xg for 5 min at RT. Pellets were resuspended in PBS for flow cytometry (FACSCalibur BD), and 10,000 events were counted. Data was analyzed using FlowJo 10.4 (Leland Stanford University) and plotted with Prism 8.01 (GraphPad Prism). To confirm the fluorescent NPs were inside the cells, confocal microscopy was performed (Zeiss LSM 700). ZFL cells were seeded on Ibidi plates (µ-Dish 35 mm) and incubated at 28°C and 5% CO₂. The next day cells at approximately 50-60% confluence were placed in minimal media. SVCV^{NP} and SVCV-IFN^{NP} at 20 µg/ml were added 2 h later and cells were incubated for 16 h at 28°C. The cells were stained with Hoechst (nuclei) and CellMask Deep Red (membrane) (Life Technologies). Images were analyzed using Imaris software v8.2.1 (Bitplane) and imaged (National Institute of Health, USA).

### Results

Modular SVCV NPs were taken up by ZFL cells. In dose-response experiments, in both, SVCV^{NP} and SVCV-IFN^{NP} cases, the mean fluorescence intensity (MFI) increased with dose until 5 - 10 µg/ml (Figures 2A and 2C) and at higher doses dropped significatively. This decrease was not related with toxicity or apoptosis since cell survival was 100% across doses and time. Possibly, the MFI had started to drop indicating cells were not able to take up more NP or there is a NP overload that had started to be metabolized, in which case the antigen is broken into small peptides which are presented by the antigen-presenting cells to other cells of the immune system. For time course experiments, a 10 µg/ml dose was chosen. In the time course experiments, at 12 and 24 h ZFL cells treated either with SVCV^{NP} or SVCV-IFN^{NP} reached the same percentage of fluorescence and the same MFI (Figures 2B and 2D). No differences in ZFL uptake at 5 or 10 µg/ml at 12 h incubation were observed between SVCV^{NP} and SVCV-IFN^{NP} when both NPs were assayed in parallel (Figure 2E). In addition, these results (Figure 2E) were highly consistent with the previous ones when the NPs were assayed separately (Figures 2A-2B, 2C and 2D).

The confocal microscopy images of SVCV^{NP} and SVCV-IFN^{NP} treated ZFL cells showed that both NPs were internalized and accumulated inside the cytosol. The 3D images demonstrated the complete internalization of both SVCV^{NP} and SVCV-IFN^{NP} in ZFL cells (data not shown). Confocal microscopy assays were performed at 20 µg/ml of NPs at 24 h and the cytometry uptake data showed that 100% and 90 % of the cells were fluorescent at 20 µg/ml. However, the Mean Fluorescent Intensity (MFI) in SVCV-IFN^{NP} treated cells (Figure 2C) was lower than in SVCV^{NP} treated cells (Figure 2A). Apparently, confocal microscopy images did not show any difference between SVCV^{NP} and SVCV-IFN^{NP} treated cells in terms of size or intensity of the fluorescent agglomerates.

### Example 3: Gene Expression Analysis in ZFL and ZF4 Stimulated with NPs

### Methods.

### RNA extraction and Q-PCR

Total RNA of ZFL cells was extracted using TriReagent (Sigma-Aldrich) following the manufacturer's instructions and RNA yield and quality was determined on Nanodrop ND-1000 (Thermo Fisher Scientific) and integrity assessed on the Agilent 2100 Bioanalyzer using RNA 6000 Nano Lab-Chip kit (Agilent Technologies). Then, cDNA was synthesized from 1 µg high quality total RNA using iScript cDNA synthesis kit (Bio-Rad). Quantitative real-time PCR (qPCR) was performed at 60°C annealing temperature in a CFX384 touch real-time PCR detection system (Bio-Rad) using iTaq Universal SYBR Green Supermix kit (Bio-Rad). Each PCR mixture consisted of 5 µl SYBR Green Supermix, 0.4 µM specific primers, 2 µl diluted cDNA and 2.6 µl water (Sigma-Aldrich) in a final volume of 10 µl. The ef1-a gene was used as a reference gene. The dilution factor for all the genes tested was 1:10 (*vig1, mx, ccl4, Imp2, ifngr1* and *ef1-α*)*.* Amplification efficiencies for primers *Imp2* and *ifngr1* were calculated. The primers of *ifngr1* are those of SEQ ID NO: 9 (TGTGAAGGCATTATGGAACGAA) for the forward primer, and SEQ ID NO: 10 (CTTCCATGTTGATTGAAGGTGAAT) for the reverse primer. The primers of *Imp2* are those of SEQ ID NO: 11 (TTATTTCCAGAACCGGGATG) for the forward primer, and SEQ ID NO: 12 (ACTCCTGGCATTCCCTCTTT) for the reverse primer. All the samples (n=6 per treatment) were run in triplicate, and data were analyzed for individual replicates using the Livak method (Livak, K.J., Schmittgen, T.D., 2001. Analysis of Relative Gene Expression Data Using Real-Time Quantitative PCR and the 2-ΔΔCT Method. Methods 25, 402-408. https://doi.ora/10.1006/meth.2001.1262).

For total RNA isolation of ZF4 cells, the E.Z.N.A.O Total RNA Kit (Omega Bio-Tek Inc., Norcross, GA, USA) was used according to the manufacturer's instructions. Then, RNA was quantified with a NanoDrop Spectrophotometer (Nanodrop Technologies, Wilmington, DE, USA), and M-MLV reverse transcriptase (Invitrogen, Thermo Fisher Scientific) was used for cDNA synthesis from RNA, as previously described (Chico, V., Gomez, N., Estepa, A., Perez, L., 2006. Rapid detection and quantitation of viral hemorrhagic septicemia virus in experimentally challenged rainbow trout by real-time RT-PCR. J Virol Methods 132, 154-159. https://doi.org/10.1016/j.jviromet.2005.10.005). To evaluate the gene expression in ZF4 cells, RT-qPCR was carried out in 20 µl reactions containing 24 ng of cDNA, 10 µl of SYBR green PCR master mix (Thermo Fischer Scientific), and a 900 nM final concentration of each primer using the QUANTSTUDIO 3 System (Applied Biosystems, Thermo Fisher Scientific Inc.). The ef1-a gene was used as a reference gene.

### Zebrafish husbandry

Wild type zebrafish were kept in a re-circulating aquarium with water temperature at 28 ± 1 °C. The lighting conditions were 14:10 h (light:dark) and adult fish were fed twice a day at a rate of 2% bodyweight. Ammonia, nitrite, nitrate and pH levels were measured once a week and were kept below toxic levels and pH at 7.5 ± 0.5. For in-tank breeding, two females and four males were transferred in groups to a breeding tank in the late afternoon. The next morning after 3 h of the onset of light embryos were collected and cultured in embryo medium (E3 medium) in a Petri dish (Deltalab). Fertilized eggs were separated from unfertilized eggs using a plastic pipette (Deltalab). All experiments involving zebrafish were performed following International Guiding Principles for Research Involving Animals (EU 2010/63) and the Ethics Committees of the Universitat Autònoma de Barcelona (UAB, CEEH) and Generalitat Valenciana (2019/VSC/PEA/0203)

### In vivo uptake in zebrafish larvae.

Groups of five larvae per treatment were distributed on 6-well plates (Thermo Fisher) in duplicate and immersed in 1 ml of E3 medium with PBS, fluorescent SVCV^{NP} and SVCV-IFN^{NP} (10 µg/ml), GFP (10 µg/ml) and LPS (10 µg/ml). Biodistribution after 24 and 48 h zebrafish larvae was evaluated using a fluorescence stereomicroscope (Nikon SMZ800) coupled with a camera (Nikon DS-Fi2). In parallel groups of n=5 larvae were treated as indicated, washed and homogenized in TriReagent for gene expression analysis as indicated in RNA extraction and Q-PCR section.

### Results

To see whether the antigenic NPs could induce an antiviral response in line with that provoked by viral Infection, ZFL cells were stimulated with the viral NPs overnight (16 h) at 10 µg/ml. Poly(I:C) (10 µg/ml) was also used as a viral dsRNA mimic, LPS as a bacterial mimic and iRFP^{NP} (10 µg/ml) as a control NP made with an immunologically irrelevant protein. The expression of genes, markers of the immune response to viral Infection and the IFN response, was tested by qPCR (Figures 3A, 3B, 3C, 3D and 3E). In ZFL cells a typical strong antiviral response after Poly(I:C) treatment was previously reported (Ruyra, A., Torrealba, D., Morera, D., Tort, L., MacKenzie, S., Roher, N., 2015. Zebrafish liver (ZFL) cells are able to mount an anti-viral response after stimulation with Poly (I:C). Comp Biochem Physiology Part B Biochem Mol Biology 182, 55-63. https://doi.org/10.1016/j.cbpb.2014.12.002). iRFP^{NP} protein without a relevant immune role does not induce the expression of antiviral genes while SVCV^{NP} and SVCV-IFN^{NP} were able to stimulate ZFL cells by increasing the gene expression of anti-viral genes such as *vig1, mx* or *ccl4*. Importantly, the inclusion of the IFNγ protein module enhanced the ability of SVCV^{NP} to stimulate an antiviral response showing that IFNγ could act as a potent adjuvant when included within the same NP (Figures 3A, 3B, 3C, 3D and 3E). Induction of the expression of IFNγ receptor (*ifngr*) and low molecular mass polypeptide *(Imp)-2* were observed after SVCV-IFN^{NP} indicating a parallel induction of IFNγ signalling pathways presumably after IFNγ binds to its receptor.

### Example 4. Uptake of SVCV^{NP} and SVCV-IFN^{NP} by zebrafish larvae

### Methods

### Zebrafish husbandry

Wild type zebrafish were kept in a re-circulating aquarium with water temperature at 28 ± 1 °C. The lighting conditions were 14:10 h (light:dark) and adult fish were fed twice a day at a rate of 2% bodyweight. Ammonia, nitrite, nitrate and pH levels were measured once a week and were kept below toxic levels and pH at 7.5 ± 0.5. For in-tank breeding, two females and four males were transferred in groups to a breeding tank in the late afternoon. The next morning after 3 h of the onset of light embryos were collected and cultured in embryo medium (E3 medium) in a Petri dish (Deltalab). Fertilized eggs were separated from unfertilized eggs using a plastic pipette (Deltalab). All experiments involving zebrafish were performed following International Guiding Principles for Research Involving Animals (EU 2010/63) and the Ethics Committees of the Universitat Autònoma de Barcelona (UAB, CEEH) and Generalitat Valenciana (2019/VSC/PEA/0203)

### In vivo uptake in zebrafish larvae

Groups of five larvae per treatment were distributed on 6-well plates (Thermo Fisher) in duplicate and immersed in 1 ml of E3 medium with PBS, fluorescent SVCV^{NP} and SVCV-IFN^{NP} (10 µg/ml), GFP (10 µg/ml) and LPS (10 µg/ml). Biodistribution after 24 and 48 h zebrafish larvae was evaluated using a fluorescence stereomicroscope (Nikon SMZ800) coupled with a camera (Nikon DS-Fi2). In parallel groups of n=5 larvae were treated as indicated, washed and homogenized in TriReagent for gene expression analysis as indicated in RNA extraction and Q-PCR section.

### Results

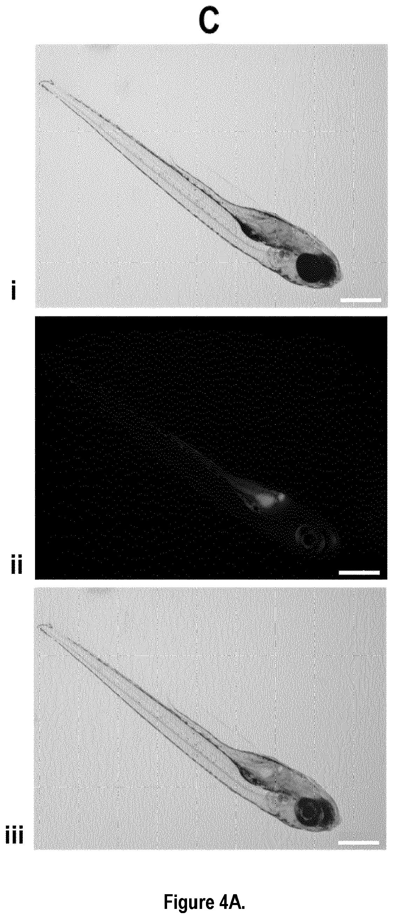

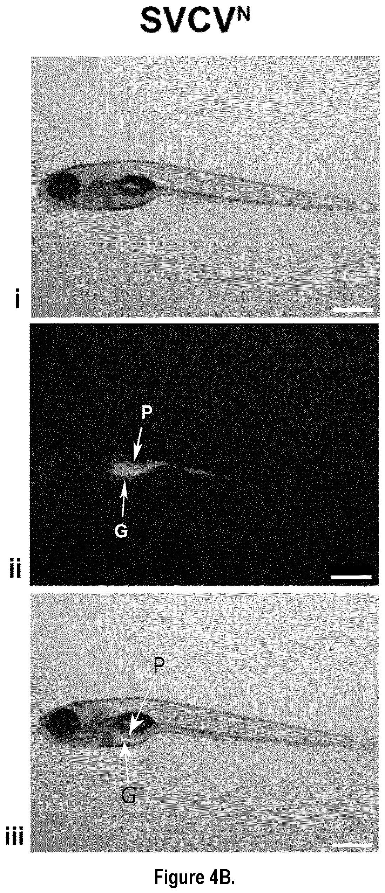

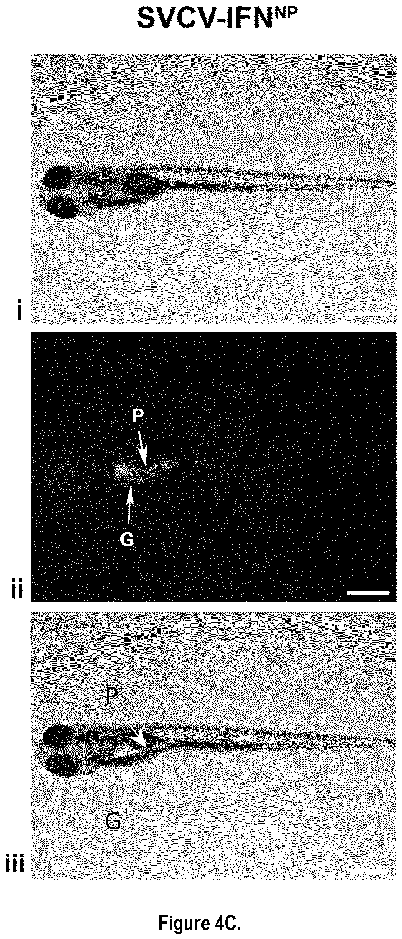

Hatched zebrafish larvae (approximately at 72 hours post fertilization (hpf)) were incubated with 10 and 20 µg/ml of fluorescent SVCV^{NP} and SVCV-IFN^{NP} in E3 medium. After 24 and 48 h living larvae were anesthetized and imaged using a stereoscopic microscope (Figures 4A, 4B and 4C). Uptake of SVCV^{NP} at 24 h was almost undetectable (n = 6 larvae) while observed high levels of fluorescence was observed at 48 h in around 100% of larvae mainly showing accumulation in the gastrointestinal tract and pancreas (Figures 4A, 4B and 4C). Importantly, SVCV^{NP} and SVCV-IFN^{NP} treated larvae died after 24 h incubation at the highest dose, 20 µg/ml. At 10 µg/ml the whole set of larvae survived after immersion in the SVCV^{NP} and SVCV-IFN^{NP} solution. Toxicity of IFNγ has been previously described in microinjected zebrafish embryos overexpressing IFNγ (López-Muñoz et al., Evolutionary conserved pro-inflammatory and antigen presentation functions of zebrafish IFNγ revealed by transcriptomic and functional analysis, Molecular Immunology, Volume 48, Issues 9-10, 2011, Pages 1073-1083, ISSN 0161-5890, https://doi.ora/10.1016/i.motimm.2011.01.015).

### Example 5. Protective effect of the vaccines against SVCV Infection in zebrafish

### Methods

Zebrafish, average size 35 mm and 0.4 g, were purchased from a local pet store. Fish were acclimatized in 20 L tanks at 21°C for 14 days before the vaccination trial. Zebrafish were divided into five groups (n=12): the non-Infected control, the SVCV-Infected control, the iRFP^{NP} control group, SVCV^{NP} group, and SVCV-IFN^{NP} group. Each individual was anesthetized with tricaine (tricaine methanesulfonate, Sigma-Aldrich) (40 mg/l) and intraperitoneally (i.p.) injected with 30 µl of PBS containing 5 µg of each antigen using 23G syringe-needle (Terumo Europe N.V.). At 30 days post-vaccination (dpv), fish were Infected by bath-immersion for 2 h in aquarium water containing SVCV (4 × 10⁴ TCID50/ml). Individuals were maintained at 21°C over the course of the challenge, and the mortality rate of each group was recorded daily.

### Results

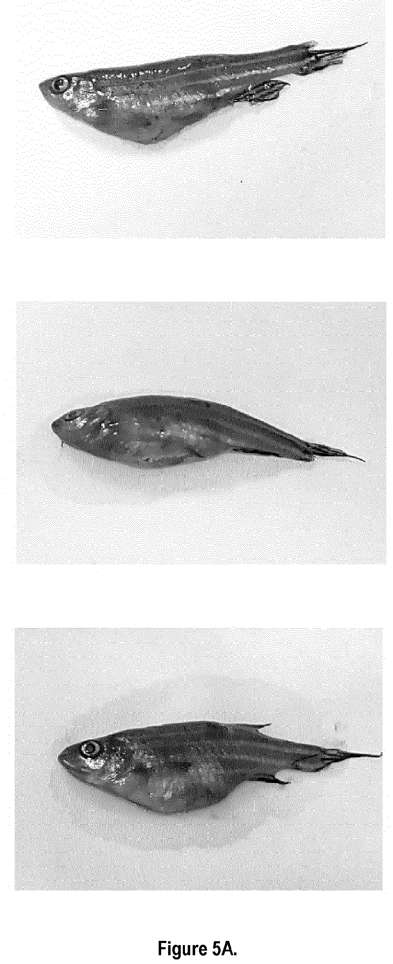

A preliminary test of NPs toxicity on adult animals was run after i.p injection of 5 and 10 µg of SVCV^{NP} and SVCV-IFN^{NP} and no signs of macrotoxicity were observed. A survival rate assay was performed on SVCV-Infected zebrafish after different nanoparticle vaccine treatments. Unvaccinated zebrafish had 83,3% cumulative percentage mortality after 16 days of Infection with SVCV. Mortality rates in the iRFP^{NP}, SVCV^{NP} and SVCV-IFN^{NP} were 41.7%, 50.0% and 20.0%, respectively (Figure 5B). The main onset of mortality occurred between 4 and 8 days post-infection (dpi), although this was delayed in the SVCV-IFN^{NP} group where first mortalities were recorded at 7 dpi. Dead fish showed clinical signs of SVCV (Figure 5A) and presence of virus in the diseased fish was confirmed by RT-qPCR.

### Example 6: Biomaterial physical-chemical features: nanosized material with extremely good resistance properties. Manufacturing proteins for inclusion in feed. (VHSV and VNNV)

The VHSV and VNNV antigens with the IFNγ module were produced in *E. coli* and purified as nanosized structures at high yield (e.g 11, 2 mg/l for VHSV-IFN^{NP}), high purity (data not shown) using a short and simple enzymatic/mechanical purification procedure, which minimizes costs and not needing further chromatographic purification steps. These biomaterials are extremely resistant to the harsh conditions of pH and temperature expected to occur during feed manufacturing (100°C temperature) and *in vivo* digestion (pH 2), thus not needing for an extra encapsulation. Same resistance against temperature and pH was found for VHSV-IFN^{NP} (SEQ ID NO: 16), VNNV-IFN^{NP} (SEQ ID NO: 17) and iRFP^{NP}.
SEQ ID NO: 17 is

SHVSARMNRTIQNLLLHYKIPPKDRFNGKPVFSREPLAGKVETKKLFMVGVLETYEKLIGQMLTHLPTPNPPTT
GIHVRLGAATAAATAPGAGGEVGKELNFILKKVQELRKHRYQEQSELLQGLEALGKIQMDNFVVQSKALWELP
WLYEEASSISNGGSSRSSMVRKGDKKLAKPATTKAANPQPRRRATQRRRSGRADAPLAKASTITGFGRATN
DVHISGMSRIAQAVVPAGTGTDGKIIVDSTIVPELLPRLGHAARIFQRYAVETLEFEIQPMCPANTGGGYVAGFL
PNPTDNDHTFDALQATRGAVVAKWWESRTVRPQYTRTLLWTSTGKEQRLTSPGRLVLLCVGSNTDVVNVSV
MCRWSVRLSVPSLETPEDTTAPITTQAPLHNDSINNGYTGFRSILLGSTQLDLAPANAVFVTDKPLPIDYSLGV
GDVDRAVYWHLRKKAGDTQVPAGYFDWGLWDDFNKTFTVGAPYYSDQQPRQILLPAGTLFTRVDSENHHH
HHH.

A vaccinal feed was developed at small scale with VHSV-IFN^{NP} using standard extrusion protocols. Once the feed was manufactured, VHSV-IFN^{NP} can be isolated and detected after delipidation by a basic extraction from food pellets and it remains intact (Figure 6).

### Example 7: Biomaterial functional features: how these biomaterials impact on fish immune system (VHSV and VNNV).

The modular system of the viral antigen together with species specific IFNγ domain (effector) is also expressed at high levels by *E. coli* BL21 (e.g 11 mg/l and 14,5 mg/l of culture for VHSV with and w/o IFNy). Functional properties of VHSV^{NP} and VHSV-IFN^{NP} have been demonstrated *in vitro, ex vivo* and *in vivo* (data not shown). Representative data of the VHSV-IFN^{NP} ability to stimulate the antiviral immune response in Antigen. The inclusion of IFNγ module provided with in-vaccine adjuvant properties in *in vitro* assays showing improved stimulatory abilities compared with the VHSV^{NP}.

Not only HKM or RTGut cell line are stimulated by VHSV-IFN^{NP} but also intraperitoneal injection of VHSV-IFN^{NP} demonstrate that fluorescent NPs could be detected in immune relevant tissue and stimulate the gene expression of immune genes in Spleen, HK and erythrocytes (RBCs) (data not shown).

Moreover, regarding the protection conferred by the NPs vaccines, promising results were obtained since the presence of specific antibodies in response to VHSV^{NP} and VHSV-IFN^{NP} injected intraperitoneally, was detected, observing higher antibody titters in VHSV-IFN^{NP} injected fish (data not shown).

Separately, a first laboratory-scale trial with VHSV-IFN^{NP} vaccinal feed showed promising protection by in-feed VHSV-IFN^{NP}. Presence of anti-VHSV-G protein specific antibodies in sera from trout immunized with in-feed VHSV^{NP} or VHSV-IFN^{NP}. Rainbow trout were fed for 20 days with iRFP^{NP} or VHSV-IFN^{NP} in-feed. After 30 days, fish sera were collected and the presence of anti-VHSV-G protein specific antibodies was analyzed and detected by ELISA using concentrated VHSV. Absorbance readings were measured at 492 and 620 nm. (Figure 7A). 60 days after end of vaccinal feed administration, rainbow trout was Infected with VHSV 1.5×10⁸ TCID50/fish, by means of intramuscular injection. Fish injected with medium were used as the control. Fish mortality was monitored daily. Fish fed with VHSV-IFN^{NP} presented lower cumulative mortality compared to iRFP^{NP} fed fish (Figure 7B).

### Citation List

### Non Patent Literature

Thwaite R, Ji J, Torrealba O, Col/ J, Sabés M, Villaverde A and Roher N (2018) Protein Nanoparticles Made of Recombinant Viral Antigens: A Promising Biomaterial for Oral Delivery of Fish Prophylactics. Front. Immunol. 9:1652. https://doi.org/10.3389/fimmu.2018.01652
Fijan, N., Petrinec, Z., Sulimanovic, D., & Zwillenberg, L.O. (1971). Isolation of the viral causative agent from the acute form of Infectious dropsy of carp. Veterinarski Arhiv, 41, 125-138
Torrealba, D., Parra, D., Seras-Franzoso, J., Vallejos-Vidal, E., Yero, D., Gibert, I., Villaverde, A., Garcia-Fruitós, E., Roher, N., 2016a. Nanostructured recombinant cytokines: A highly stable alternative to short-lived prophylactics. Biomaterials 107, 102-114. https://doi.org/10.1016/j.biomaterials.2016.08.043
Seras-Franzoso, J., Sánchez-Chardi, A., Garcia-Fruitós, E., Vázquez, E., Villaverde, A., 2016. Cellular uptake and intracellular fate of protein releasing bacterial amyloids in mammalian cells. Soft Matter 12, 3451-3460. https://doi.orq/10.1039/c5sm02930a
Livak, K.J., Schmittgen, T.D., 2001. Analysis of Relative Gene Expression Data Using Real-Time Quantitative PCR and the 2-ΔΔCT Method. Methods 25, 402-408. https:/Idoi.orq/10.1006/meth.2001.1262
Chico, V., Gomez, N., Estepa, A., Perez, L., 2006. Rapid detection and quantitation of viral hemorrhagic septicemia virus in experimentally challenged rainbow trout by real-time RT-PCR. J Virol Methods 132, 154-159. https://doi.orq/10.1016/i.iviromet.2005.10.005
Ruyra, A., Torrealba, D., Morera, D., Tort, L., MacKenzie, S., Roher, N., 2015. Zebrafish liver (ZFL) cells are able to mount an anti-viral response after stimulation with Poly (I:C). Comp Biochem Physiology Part B Biochem Mol Biology 182, 55-63. https://doi.org/10.1016/j.cbpb.2014.12.002
López-Muñoz et al., Evolutionary conserved pro-inflammatory and antigen presentation functions of zebrafish IFNγ revealed by transcriptomic and functional analysis, Molecular Immunology, Volume 48, Issues 9-10, 2011, Pages 1073-1083, ISSN 0161-5890, https://doi.org/10.1016/j.molimm.2011.01.015

## Claims

1. A polypeptide comprising the amino acid sequence of an interferon or a fragment thereof, the amino acid sequence of a viral antigen or a fragment thereof and, optionally, a linker between them.

2. The polypeptide according to claim 1, wherein the polypeptide has formula (I):
R1-(L)n-R2 (I)
wherein:
n is an integer selected from 0 or 1;
R1, which is at the N-terminal end of the polypeptide, comprises the amino acid sequence of an interferon or a fragment thereof;
L is a linker; and
R2, which is at the C-terminal end of the polypeptide, comprises the amino acid sequence of a viral antigen or a fragment thereof.

3. - The polypeptide according to any one of claims 1-2, wherein R2 is the amino acid sequence of a viral antigen of a virus causing disease in aquatic animals selected from fish or teleost and crustacean.

4. - The polypeptide according to any one of claims 1-3, wherein R2 the amino acid sequence of a viral antigen of a virus causing disease, said virus selected from Infectious pancreatic necrosis virus (IPNV), Viral Hemorrhagic Septicemia Virus (VHSV), Viral Nervous Necrosis Virus (VNNV) and Spring Viraemia of Carp Virus (SVCV).

5. - The polypeptide according to claim 4, wherein the virus is selected from SVCV and VHSV.

6. - The polypeptide according to any one of claims 1-5, wherein R2 comprises the amino acid sequence as set forth in SEQ ID NO: 1, or a sequence at least 85% identical to SEQ ID NO: 1; or alternatively wherein R2 comprises the amino acid sequence as set forth in SEQ ID NO: 5, or a sequence at least 85% identical to SEQ ID NO: 5.

7. - The polypeptide according to any one of claims 1-6, wherein R1 comprises the amino acid sequence of an interferon or a fragment thereof of an animal species selected from fish or teleost and crustacean, the interferon selected from the group consisting of interferons Type I and Type II.

8. - The polypeptide according to any one of claims 1-7, wherein R1 comprises the amino acid sequence of the interferon gamma-1.

9. - The polypeptide according to any one of claims 1-8, wherein R1 comprises the amino acid sequence as set forth in SEQ ID NO: 2 or a sequence at least 85% identical to SEQ ID NO: 2.

10. - The polypeptide according to any one of claims 1-9, wherein n is 1, and the linker L is a peptide comprising SEQ ID NO: 3 (GGSSRSS).

11. - A protein nanopellet comprising one or more of the polypeptides as defined in any one of claims 1-10, and with a particle diameter from 400 nm to 1500 nm, measured by electron microscopy.

12. - An immunogenic composition comprising one or more polypeptides as defined in any one of claims 1-10, and/or the protein nanopellet as defined in claim 11.

13. - A vaccine comprising the immunogenic composition as defined in claim 12, and a pharmaceutically acceptable excipient and/or carrier.

14. - A polypeptide as defined in any one of claims 1-10, and/or the protein nanopellet as defined in claim 11, and/or the immunogenic composition as defined in claim 12, and/or the vaccine as defined in claim 13, for use in therapy, in particular, for the prevention and/or treatment of a viral disease.

15. - A food composition comprising a polypeptide as defined in any one of claims 1-10, and/or the protein nanopellet as defined in claim 11, and/or the immunogenic composition as defined in claim 12, and/or the vaccine as defined in claim 13.
